# EUROPEAN PATENT APPLICATION

(11) **EP 4 520 770 A1**
(43) Date of publication of application: **12.03.2025**
(21) Application number: 23799659.0
(22) Date of filing: 02.05.2023
(51) Int. Cl.: C07K 16/28, A61P 25/28, A61K 39/00

(54) **ANTI-CD300C ANTIBODY OR ANTIGEN-BINDING FRAGMENT THEREOF, AND USES THEREOF FOR PREVENTING OR TREATING NEURODEGENERATIVE BRAIN DISEASE**

(30) Priority: 02.05.2022 KR 20220054434
(71) Applicant: CentricsBio, Inc., Seoul 05836 (KR)
(72) Inventor: JEON, Jae-Won, Suwon-si Gyeonggi-do 16512 (KR); LEE, Suin, Gunpo-si Gyeonggi-do 15801 (KR); KIM, Haneul, Namyangju-si Gyeonggi-do 12221 (KR)
(74) Representative: Regimbeau
(86) International application number: PCT/KR2023/005995
(87) International publication number: WO 2023/214778

(57) **Abstract**

The present invention relates to a pharmaceutical composition for preventing or treating degenerative brain disease, comprising one or more of an anti-CD300c antibody and an antigen-binding fragment thereof; a method for preventing or treating degenerative brain disease using the same; a feed composition for preventing or improving degenerative brain disease, comprising one or more of an anti-CD300c antibody and an antigen-binding fragment thereof; a food composition; and the use of the anti-CD300c antibody and an antigen-binding fragment thereof for preventing or treating degenerative brain disease.

## Description

### [Technical Field]

The present invention relates to a pharmaceutical composition for preventing or treating degenerative brain disease, comprising one or more of an anti-CD300c antibody and an antigen-binding fragment thereof; a method for preventing or treating degenerative brain disease using the same; a feed composition or food composition for preventing or treating degenerative brain disease, comprising one or more of an anti-CD300c antibody and an antigen-binding fragment thereof; and use of the anti-CD300c antibody and/or an antigen-binding fragment thereof in preventing or treating degenerative brain disease.

### [Background Art]

Alzheimer's disease (AD) is a representative example of a degenerative brain disease characterized by progressive loss of cognitive and memory functions and neurodegeneration of the central nervous system, accounting for approximately 50% of dementia cases. AD is characterized by the presence of amyloid plaques, neurofibrillary tangles, synaptic loss, selective neuronal cell death, *etc.* Amyloid plaques are induced by extracellular amyloid-beta peptides at abnormal levels, while neurofibrillary tangles are associated with the presence of intracellular hyperphosphorylated tau proteins. AD symptoms are characterized by amnesia, along with dysphasia, dyspraxia, and agnosia (the inability to recognize objects, people, sounds, shapes, or smells), which contribute to the correlation with cortical association areas. The incidence of AD increases particularly after the age of 65 and is rapidly increasing worldwide as the population ages.

Commercially available AD treatments help improve cognitive function but primarily consist of AChE inhibitors and NMDA receptor antagonists which target temporary symptom relief. Aducanumab, a monoclonal antibody drug targeting amyloid-beta, received FDA approval in 2021. Unlike the existing symptom-improving drugs, Aducanumab targets the fundamental pathophysiology, but there is controversy regarding its efficacy, side effects, *etc.* Additionally, a study of targeting PD-1/PD-L1 signaling process to treat AD has been reported (Non-Patent Literature 1). This study found that blocking PD-1/PD-L1 signaling resulted in increased monocyte-derived macrophages within the brain parenchyma, and finally suggested the possibility of an immunological method through the reduction of plaques and brain lesions. Development of AD treatments is highly challenging with a very low success rate; however, with the acceleration in population aging, the increase in the number of AD patients, and the growth in the socioeconomic costs associated with AD, there is a need for the development of various fundamental treatments rather than symptom-relieving drugs.

Meanwhile, the CD300c (CD300 antigen-like family member C) protein is a protein encoded by the CD300c gene in humans, which is present on the surface of various tumor cells and monocytes. It has been revealed that by inhibiting the activity or expression of the CD300c protein, the proliferation of cancer cells can be reduced through the improvement in immune functions (*e.g.,* T cell activation) (Patent Literature 1).

(Patent Literature 1) Korean Patent Application Publication No. 10-2021-0060355
(Non-Patent Literature 1) 1. Rosenzweig, N., Dvir-Szternfeld, R., Tsitsou-Kampeli, A., Keren-Shaul, H., Ben-Yehuda, H., Weill-Raynal, P., Cahalon, L., Kertser, A., Baruch, K., Amit, I., Weiner, A., & Schwartz, M. PD-1/PD-L1 checkpoint blockade harnesses monocyte-derived macrophages to combat cognitive impairment in a tauopathy mouse model. Nature Communications, 10(1) (2019).

### [Detailed Description of the Invention]

### [Technical Problem]

An object of the present invention is to provide a pharmaceutical composition for preventing or treating degenerative brain disease, comprising one or more of an anti-CD300c antibody and an antigen-binding fragment thereof; a method for preventing or treating degenerative brain disease using the same; a feed composition or food composition for preventing or improving degenerative brain disease, comprising one or more of an anti-CD300c antibody and an antigen-binding fragment thereof; and a use of the anti-CD300c antibody and/or an antigen-binding fragment thereof for preventing or treating degenerative brain disease.

### [Technical Solution]

An object of the present invention is to resolve all the aforementioned problems.

Another object of the present invention is to provide an antibody drug for preventing or treating degenerative brain disease.

Still another object of the present invention is to provide a composition for preventing or treating degenerative brain disease.

Still another object of the present invention is to provide a method for preventing or treating degenerative brain disease.

Still another object of the present invention is to provide a use of the anti-CD300c antibody and an antigen-binding fragment thereof in preventing or treating degenerative brain disease.

### [Advantageous Effects]

According to the present invention, an antibody that specifically binds to CD300c and an antigen-binding fragment thereof may be effectively used as a preventive or therapeutic agent for degenerative brain disease.

Through a Y-maze test and a Morris water maze test using 5xFAD mice that have been genetically modified to express genes related to degenerative brain disease, it was confirmed that the anti-CD300c antibody or an antigen-binding fragment thereof can significantly restore the cognitive and memory functions of the degenerative brain disease mouse models. Therefore, the anti-CD300c antibody or an antigen-binding fragment thereof has effective drug efficacy for preventing or treating degenerative brain disease.

### [Brief Description of the Drawing]

FIG. 1 shows the results of confirming the binding affinity of anti-CD300c monoclonal antibodies to the CD300c antigen using binding ELISA.
FIG. 2 shows the sequence comparison results among the heavy chain variable regions of the anti-CD300c monoclonal antibodies CB201, CL10, and SL18.
FIG. 3 shows the results of confirming the binding affinity of the anti-CD300c monoclonal antibody to the CD300c antigen using surface plasmon resonance (SPR).
FIG. 4 shows the results of confirming the binding affinity of an anti-CD300c monoclonal antibody to CD300c antigens on cells.
FIG. 5 shows the results of confirming the promotion of monocyte-derived macrophage (MDM) differentiation by an anti-CD300c monoclonal antibody by the observation of cell morphology.
FIG. 6 shows the results of confirming the promotion of differentiation into MDM by an anti-CD300c monoclonal antibody through an analysis of factors involved in cell signaling.
FIG. 7 shows the results of confirming the promotion of differentiation into MDM by the anti-CD300c monoclonal antibody CB201 through the measurement of the production of differentiation markers (TNF-α, IL-1β, and IL-8).
FIG. 8 shows the results of confirming the promotion of differentiation into MDM by the anti-CD300c monoclonal antibodies CL10 and SL18 through the measurement of the production of a differentiation marker (TNF-α).
FIG. 9 shows the results of confirming the promotion of differentiation into MDM by anti-CD300c monoclonal antibodies through an analysis of extracellular protein markers.
FIG. 10 shows the results of confirming the promotion of differentiation into MDM by an anti-CD300c monoclonal antibody through marker analysis (*in vivo*). Examples of the CD206+ region in the αPD-1 diagram and the iNOS+ region in the CB201 diagram are indicated by arrows in each diagram.
FIG. 11 shows the results of confirming the promotion of differentiation into MDM by an anti-CD300c monoclonal antibody through marker analysis (*in vivo*).
FIG. 12 shows the results of confirming the changes in promotion of differentiation into MDM and immune system by an anti-CD300c monoclonal antibody through nano-string immune profiling.
FIG. 13 shows a schematic diagram illustrating the animal model preparation and experimental methods for the Y-maze test.
FIG. 14 shows images from a video recorded during the Morris water maze test.
FIG. 15 shows a schematic diagram illustrating the quantification of the results of the Y-maze test and the Morris water maze test.

### [Detailed Description of Preferred Embodiments]

The present invention will be described in detail as follows. Meanwhile, each description and embodiment disclosed in this invention may also be applied to other descriptions and embodiments. That is, all combinations of various elements disclosed in this invention fall within the scope of the present disclosure. Further, the scope of the present invention is not limited by the specific description described below. Furthermore, a number of papers and patent documents are referenced and cited throughout this specification. The disclosures of the cited papers and patent documents are incorporated herein by reference in their entirety to further clarify the level of art and the description of the present invention.

An aspect of the present invention provides a pharmaceutical composition for preventing or treating degenerative brain disease, comprising one or more of an anti-CD300c antibody and an antigen-binding fragment thereof as active ingredients.

In one embodiment, the antibody of the present invention may have the ability to promote differentiation of monocytes into monocyte-derived macrophage (MDM) or M1 macrophage. This promoting ability may be enhanced compared to general treatments and immunotherapy for degenerative brain disease, but is not limited thereto. In the present invention, MDM may be M1-MDM.

In the present invention, an anti-CD300c antibody and an antigen-binding fragment thereof refer to antigen-binding molecules that can specifically bind to the CD300c protein, which may inhibit the reaction of CD300c, but are not limited thereto.

In the present invention, the term "CD300c" is used interchangeably with "CD300c protein" or "CD300c antigen," and refers to a member of the CD300 family, which are cell surface proteins involved in immune regulation. It may be expressed on antigen-presenting cells, cancer cells, or immune cells, but is not limited thereto.

In the present invention, the term "antibody" refers to a protein molecule that can specifically recognize an antigenic site, including an immunoglobulin that has immunological reactivity with a specific antigen or a part thereof. The antibody of the present invention includes polyclonal antibodies, monoclonal antibodies, whole antibodies, and antibody fragments. Additionally, the antibody of the present invention, which includes mouse, human, rabbit, rat antibodies, *etc.,* is not limited by its origin. The antibody of the present invention includes animal antibodies, chimeric antibodies (*e.g.,* humanized murine antibodies), humanized antibodies, multispecific antibodies, bivalent or bispecific molecules (*e.g.,* bispecific antibodies), diabodies, triabodies, tetrabodies, minibodies, and antibody conjugates (*e.g.,* conjugates of antibodies with (poly)peptides or compounds). The antibody of the present invention further includes single-chain antibodies, scFvs, derivatives of antibody constant regions, and artificial antibodies based on protein scaffolds, which retain binding functionality to FcRn (a neonatal Fc receptor). Whole antibodies have a structure comprising two full-length light chains (LC) and two full-length heavy chains (HC), in which each light chain may be connected to a heavy chain via disulfide bonds. These whole antibodies include any antibodies such as IgA, IgD, IgE, IgM, and IgG, in which IgG includes subtypes IgG1, IgG2, IgG3, and IgG4. Such antibodies may be produced by cloning each gene into an expression vector according to conventional methods to obtain the protein encoded by the marker gene, and then manufacturing the obtained protein by conventional methods, but the methods are not limited thereto.

In the present invention, the prefix "anti-", when related to an antigen, means that the antibody is reactive with the corresponding antigen. Antibodies reactive with a specific antigen may be prepared by synthetic and/or recombinant methods such as screening recombinant antibody libraries in phage- or similar vectors, or by immunizing animals to the antigen or antigen-coding nucleic acids, but the methods are not limited thereto.

In one embodiment, the anti-CD300c antibody of the present invention may refer to an antibody reactive with CD300c.

In the present invention, the term "fragment" or "antibody fragment" refers to any portion of an antibody; scFv, dsFv, Fab, Fab', F(ab')2, Fc, Fd, sdAb, nanobody, *etc.* as well as combinations thereof, may correspond to an antibody fragment. The antibody fragment may include antigen-recognition sites but are not limited thereto.

In the present invention, the term "antigen-binding fragment" refers to a fragment that retains antigen-binding functionality. In the present invention, the antigen-binding fragment may be a fragment including a region capable of recognizing an antigenic site.

In the present invention, Fd refers to a portion of the heavy chain included in the Fab fragment. In the present invention, Fab has a structure comprising light chain and heavy chain variable regions, a light chain constant region, and a first constant region (a CH1 domain) of the heavy chain, and has one antigen-binding site. Fab' differs from Fab in that it includes a hinge region comprising one or more cysteine residues at the C-terminus of the CH1 domain of the heavy chain. An F(ab')2 antibody is formed when the cysteine residues in the hinge region of Fab' form disulfide bonds. Fv (a variable fragment) refers to the smallest antibody fragment containing only the heavy and light chain variable regions. Disulfide-stabilized Fv (dsFv) has the heavy and light chain variable regions connected by disulfide bonds, while scFv (single-chain variable fragment) typically has the heavy and light chain variable regions connected by a peptide linker. dsFv may further have heavy and light chain variable regions connected to scFv by disulfide bonds. The sdAb and nanobody are single variable domain antibody fragments, which may include, for example, an antibody fragment prepared by protein hydrolysis of or through the use of genetic recombination techniques on the variable domains of a heavy chain antibody comprising a naturally occurring single variable domain (VH) and two constant domains (CH2 and CH3); or a single domain antibody fragment prepared by artificially modifying the heavy or light chain variable domain of the antibody, but are not limited thereto. Such antibody fragments may be obtained using proteases (*e.g.,* Fab can be obtained using papain digestion, or F(ab')2 fragment can be obtained using pepsin digestion) or produced through genetic recombination techniques, but are not limited thereto.

In one embodiment, the anti-CD300c antibody of the present invention may be a monoclonal antibody.

In the present invention, the term "monoclonal antibody" refers to an antibody molecule of a single molecular composition obtained from a substantially identical population of antibodies, and such monoclonal antibodies exhibit single binding specificity and affinity for a particular epitope.

Generally, an antibody has heavy chains and light chains, each of which includes a constant region and a variable region. The variable regions of the light and heavy chains each include three hypervariable regions, known as complementarity-determining regions (CDRs), and four framework regions (FRs).

The CDRs primarily function to bind to the epitope of an antigen. The CDRs of each chain are typically referred to as CDR1, CDR2, and CDR3, sequentially from the N-terminus, and are also identified by the chain in which they are located. The CDRs are interspersed among regions referred to as invariable regions (FR), which are relatively conserved. The heavy chain variable regions (VH) and the light chain variable regions (VL) each consist of three CDRs and four FRs, arranged in the following order from the amino-terminus to the carboxy-terminus: FR1, CDR1, FR2, CDR2, FR3, CDR3, and FR4. The variable regions of the heavy and light chains contain binding domains that interact with an antigen. The CDRs of the heavy chain variable region may be referred to as HCDR1, HCDR2, and HCDR3, while the CDRs of the light chain variable region may be referred to as LCDR1, LCDR2, and LCDR3. The FRs of the heavy chain variable region may be referred to as HFR1, HFR2, HFR3, and HFR4, and the FRs of the light chain variable region may be referred to as LFR1, LFR2, LFR3, and LFR4.

In one embodiment, one or more of the antibody and an antigen-binding fragment thereof of the present invention may include:
(i) a heavy chain variable region, comprising: a CDR1 consisting of an amino acid sequence selected from the group consisting of SEQ ID NO: 7, SEQ ID NO: 19, SEQ ID NO: 31, SEQ ID NO: 43, SEQ ID NO: 55, SEQ ID NO: 67, SEQ ID NO: 79, SEQ ID NO: 91, SEQ ID NO: 103, SEQ ID NO: 115, SEQ ID NO: 127, SEQ ID NO: 139, SEQ ID NO: 151, SEQ ID NO: 163, SEQ ID NO: 175, SEQ ID NO: 187, SEQ ID NO: 199, SEQ ID NO: 211, SEQ ID NO: 223, SEQ ID NO: 235, SEQ ID NO: 247, SEQ ID NO: 259, SEQ ID NO: 271, SEQ ID NO: 283, and SEQ ID NO: 295; a CDR2 consisting of an amino acid sequence selected from the group consisting of SEQ ID NO: 8, SEQ ID NO: 20, SEQ ID NO: 32, SEQ ID NO: 44, SEQ ID NO: 56, SEQ ID NO: 68, SEQ ID NO: 80, SEQ ID NO: 92, SEQ ID NO: 104, SEQ ID NO: 116, SEQ ID NO: 128, SEQ ID NO: 140, SEQ ID NO: 152, SEQ ID NO: 164, SEQ ID NO: 176, SEQ ID NO: 188, SEQ ID NO: 200, SEQ ID NO: 212, SEQ ID NO: 224, SEQ ID NO: 236, SEQ ID NO: 248, SEQ ID NO: 260, SEQ ID NO: 272, SEQ ID NO: 284, and SEQ ID NO: 296; and a CDR3 consisting of an amino acid sequence selected from the group consisting of SEQ ID NO: 9, SEQ ID NO: 21, SEQ ID NO: 33, SEQ ID NO: 45, SEQ ID NO: 57, SEQ ID NO: 69, SEQ ID NO: 81, SEQ ID NO: 93, SEQ ID NO: 105, SEQ ID NO: 117, SEQ ID NO: 129, SEQ ID NO: 141, SEQ ID NO: 153, SEQ ID NO: 165, SEQ ID NO: 177, SEQ ID NO: 189, SEQ ID NO: 201, SEQ ID NO: 213, SEQ ID NO: 225, SEQ ID NO: 237, SEQ ID NO: 249, SEQ ID NO: 261, SEQ ID NO: 273, SEQ ID NO: 285, and SEQ ID NO: 297; and
(ii) a light chain variable region, comprising: a CDR1 consisting of an amino acid sequence selected from the group consisting of SEQ ID NO: 10, SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 142, SEQ ID NO: 154, SEQ ID NO: 166, SEQ ID NO: 178, SEQ ID NO: 190, SEQ ID NO: 202, SEQ ID NO: 214, SEQ ID NO: 226, SEQ ID NO: 238, SEQ ID NO: 250, SEQ ID NO: 262, SEQ ID NO: 274, SEQ ID NO: 286, and SEQ ID NO: 298; a CDR2 consisting of an amino acid sequence selected from the group consisting of SEQ ID NO: 11, SEQ ID NO: 23, SEQ ID NO: 35, SEQ ID NO: 47, SEQ ID NO: 59, SEQ ID NO: 71, SEQ ID NO: 83, SEQ ID NO: 95, SEQ ID NO: 107, SEQ ID NO: 119, SEQ ID NO: 131, SEQ ID NO: 143, SEQ ID NO: 155, SEQ ID NO: 167, SEQ ID NO: 179, SEQ ID NO: 191, SEQ ID NO: 203, SEQ ID NO: 215, SEQ ID NO: 227, SEQ ID NO: 239, SEQ ID NO: 251, SEQ ID NO: 263, SEQ ID NO: 275, SEQ ID NO: 287, and SEQ ID NO: 299; and a CDR3 consisting of an amino acid sequence selected from the group consisting of SEQ ID NO: 12, SEQ ID NO: 24, SEQ ID NO: 36, SEQ ID NO: 48, SEQ ID NO: 60, SEQ ID NO: 72, SEQ ID NO: 84, SEQ ID NO: 96, SEQ ID NO: 108, SEQ ID NO: 120, SEQ ID NO: 132, SEQ ID NO: 144, SEQ ID NO: 156, SEQ ID NO: 168, SEQ ID NO: 180, SEQ ID NO: 192, SEQ ID NO: 204, SEQ ID NO: 216, SEQ ID NO: 228, SEQ ID NO: 240, SEQ ID NO: 252, SEQ ID NO: 264, SEQ ID NO: 276, SEQ ID NO: 288, and SEQ ID NO: 300.

In one embodiment, in the entire amino acid sequence of the heavy chain variable region of the anti-CD300c antibody or an antigen-binding fragment thereof of the present invention, the amino acid sequence from the first amino acid to the amino acid before CDR1 may be FR1 of the heavy chain variable region; the amino acid sequence from the amino acid after CDR1 to the amino acid before CDR2 may be FR2 of the heavy chain variable region; the amino acid sequence from the amino acid after CDR2 to the amino acid before CDR3 may be FR3 of the heavy chain variable region; and the sequence from the amino acid after CDR3 to the last amino acid of the entire amino acid sequence of the heavy chain variable region may be FR4 of the heavy chain variable region. In the entire amino acid sequence of the light chain variable region of the anti-CD300c antibody or an antigen-binding fragment thereof of the present invention, the amino acid sequence from the first amino acid to the amino acid before CDR1 may be FR1 of the light chain variable region; the amino acid sequence from the amino acid after CDR1 to the amino acid before CDR2 may be FR2 of the light chain variable region; the amino acid sequence from the amino acid after CDR2 to the amino acid before CDR3 may be FR3 of the light chain variable region; and the sequence from the amino acid after CDR3 to the last amino acid of the entire amino acid sequence of the light chain variable region may be FR4 of the light chain variable region. Additionally, the polynucleotides of FR1 to FR4 of the heavy chain variable region and the polynucleotides of FR1 to FR4 of the light chain variable region can be defined similarly to the amino acid sequences. Thus, in the antibody of the present invention, the amino acid sequences of FR1 to FR4 of the heavy chain variable region and FR1 to FR4 of the light chain variable region, and the polynucleotide sequences encoding the same can be identified from SEQ ID NO: 1 to SEQ ID NO: 400.

In another embodiment, one or more of the antibody and an antigen-binding fragment thereof of the present invention may include (i) a heavy chain variable region comprising: CDR1 consisting of an amino acid sequence selected from the group consisting of SEQ ID NO: 79, SEQ ID NO: 115, and SEQ ID NO: 211; CDR2 consisting of an amino acid sequence selected from the group consisting of SEQ ID NO: 80, SEQ ID NO: 116, and SEQ ID NO: 212; and CDR3 consisting of an amino acid sequence selected from the group consisting of SEQ ID NO: 81, SEQ ID NO: 117, and SEQ ID NO: 213; and (ii) a light chain variable region comprising: CDR1 consisting of an amino acid sequence selected from the group consisting of SEQ ID NO: 82, SEQ ID NO: 118, and SEQ ID NO: 214; CDR2 consisting of an amino acid sequence selected from the group consisting of SEQ ID NO: 83, SEQ ID NO: 119, and SEQ ID NO: 215; and CDR3 consisting of an amino acid sequence selected from the group consisting of SEQ ID NO: 84, SEQ ID NO: 120, and SEQ ID NO: 216.

In one embodiment of the previously described embodiments, one or more of the antibody and an antigen-binding fragment thereof of the present invention may be selected from the following (i) to (iii): (i) one which includes: a heavy chain variable region comprising CDR1 consisting of SEQ ID NO: 79, CDR2 consisting of SEQ ID NO: 80, and CDR3 consisting of SEQ ID NO: 81; and a light chain variable region comprising CDR1 consisting of SEQ ID NO: 82, CDR2 consisting of SEQ ID NO: 83, and CDR3 consisting of SEQ ID NO: 84; (ii) one which includes: a heavy chain variable region comprising CDR1 consisting of SEQ ID NO: 115, CDR2 consisting of SEQ ID NO: 116, and CDR3 consisting of SEQ ID NO: 117; and a light chain variable region comprising CDR1 consisting of SEQ ID NO: 118, CDR2 consisting of SEQ ID NO: 119, and CDR3 consisting of SEQ ID NO: 120; and (iii) one which includes: a heavy chain variable region comprising CDR1 consisting of SEQ ID NO: 211, CDR2 consisting of SEQ ID NO: 212, and CDR3 consisting of SEQ ID NO: 213; and a light chain variable region comprising CDR1 consisting of SEQ ID NO: 214, CDR2 consisting of SEQ ID NO: 215, and CDR3 consisting of SEQ ID NO: 216.

In one embodiment of the previously described embodiments, one or more of the antibody and an antigen-binding fragment thereof of the present invention may include (i) a heavy chain variable region comprising CDR1 consisting of an amino acid sequence represented by SEQ ID NO: 79, CDR2 consisting of an amino acid sequence represented by SEQ ID NO: 80, and CDR3 consisting of an amino acid sequence represented by SEQ ID NO: 81; and (ii) a light chain variable region comprising CDR1 consisting of an amino acid sequence represented by SEQ ID NO: 82, CDR2 consisting of an amino acid sequence represented by SEQ ID NO: 83, and CDR3 consisting of an amino acid sequence represented by SEQ ID NO: 84.

In one embodiment, one or more of the antibody and an antigen-binding fragment thereof of the present invention may include a heavy chain variable region consisting of any one of SEQ ID NO: 303, SEQ ID NO: 307, SEQ ID NO: 311, SEQ ID NO: 315, SEQ ID NO: 319, SEQ ID NO: 323, SEQ ID NO: 327, SEQ ID NO: 331, SEQ ID NO: 335, SEQ ID NO: 339, SEQ ID NO: 343, SEQ ID NO: 347, SEQ ID NO: 351, SEQ ID NO: 355, SEQ ID NO: 359, SEQ ID NO: 363, SEQ ID NO: 367, SEQ ID NO: 371, SEQ ID NO: 375, SEQ ID NO: 379, SEQ ID NO: 383, SEQ ID NO: 387, SEQ ID NO: 391, SEQ ID NO: 395, and SEQ ID NO: 399; and a light chain variable region consisting of any one of SEQ ID NO: 304, SEQ ID NO: 308, SEQ ID NO: 312, SEQ ID NO: 316, SEQ ID NO: 320, SEQ ID NO: 324, SEQ ID NO: 328, SEQ ID NO: 332, SEQ ID NO: 336, SEQ ID NO: 340, SEQ ID NO: 344, SEQ ID NO: 348, SEQ ID NO: 352, SEQ ID NO: 356, SEQ ID NO: 360, SEQ ID NO: 364, SEQ ID NO: 368, SEQ ID NO: 372, SEQ ID NO: 376, SEQ ID NO: 380, SEQ ID NO: 384, SEQ ID NO: 388, SEQ ID NO: 392, SEQ ID NO: 396, and SEQ ID NO: 400, but are not limited thereto.

In one embodiment of the previously described embodiments, one or more of the anti-CD300c antibody and an antigen-binding fragment thereof may include a heavy chain variable region consisting of any one of SEQ ID NO: 327, SEQ ID NO: 339, and SEQ ID NO: 371; and a light chain variable region consisting of any one of SEQ ID NO: 328, SEQ ID NO: 340, and SEQ ID NO: 372, but are not limited thereto.

In one embodiment of the previously described embodiments, one or more of the anti-CD300c antibody and an antigen-binding fragment thereof may include a heavy chain variable region consisting of SEQ ID NO: 327 and a light chain variable region consisting of SEQ ID NO: 328, but are not limited thereto.

In one embodiment, one or more of the heavy chain variable regions of the anti-CD300c antibody and an antigen-binding fragment thereof of the present invention may be encoded by any one of SEQ ID NO: 305, SEQ ID NO: 309, SEQ ID NO: 313, SEQ ID NO: 317, SEQ ID NO: 321, SEQ ID NO: 325, SEQ ID NO: 329, SEQ ID NO: 333, SEQ ID NO: 337, SEQ ID NO: 341, SEQ ID NO: 345, SEQ ID NO: 349, SEQ ID NO: 353, SEQ ID NO: 357, SEQ ID NO: 361, SEQ ID NO: 365, SEQ ID NO: 369, SEQ ID NO: 373, SEQ ID NO: 377, SEQ ID NO: 381, SEQ ID NO: 385, SEQ ID NO: 389, SEQ ID NO: 393, SEQ ID NO: 397, and SEQ ID NO: 341, and one or more of the light chain variable region of the anti-CD300c antibody and an antigen-binding fragment thereof of the present invention may be encoded by any one of SEQ ID NO: 306, SEQ ID NO: 310, SEQ ID NO: 314, SEQ ID NO: 318, SEQ ID NO: 322, SEQ ID NO: 326, SEQ ID NO: 330, SEQ ID NO: 334, SEQ ID NO: 338, SEQ ID NO: 342, SEQ ID NO: 346, SEQ ID NO: 350, SEQ ID NO: 354, SEQ ID NO: 358, SEQ ID NO: 362, SEQ ID NO: 366, SEQ ID NO: 370, SEQ ID NO: 374, SEQ ID NO: 378, SEQ ID NO: 382, SEQ ID NO: 386, SEQ ID NO: 390, SEQ ID NO: 394, SEQ ID NO: 398, and SEQ ID NO: 402, but are not limited thereto.

In another embodiment, one or more of the antibody and an antigen-binding fragment thereof of the present invention may include a heavy chain variable region comprising CDR1 to CDR3 consisting of amino acid sequences respectively represented by the following formulas (1) to (3), and a light chain variable region comprising CDR1 to CDR3 consisting of amino acid sequences respectively represented by the following formulas (4) to (6):

FTFX1X2X3X4MX5WVR (1)

wherein,
X1 = G or S;
X2 = S, R, or D;
X3 = N or Y;
X4 = Y, A, G, or H; and
X5 = S or H;

X1ISX2SGX3X4TYYAX5 (2)

wherein,
X1 = T or A;
X2 = G or S;
X3 = T or G;
X4 = S or Y; and
X5 = D or E;

YCAX1X2X3X4X5X6X7X8X9W (3)

wherein,
X1 = R or S;
X2 = G or S;
X3 = M, S, Y, or I;
X4 = W, Q, G, or R;
X5 = G or L;
X6 = M, I, or P;
X7 = D, F, or L;
X8 = V or D; and
X9 = I, Y, or absent;

CX1X2X3X4X5X6X7X8X9X10X11VX12W (4)

wherein,
X1 = T or S;
X2 = G or R;
X3 = K, N, or S;
X4 = H, N, or S;
X5 = R, I, or G;
X6 = H, G, or I;
X7 = T, I, or S;
X8 = R, A, K, or absent;
X9 = R, S, G, or absent;
X10 = N or absent;
X11 = Y or absent; and
X12 = N, H, or Q;

X1X2X3X4RPSGVX5 (5)

wherein,
X1 = L, S, R, or E;
X2 = D, K, or N;
X3 = S or N;
X4 = E, N, Q, or K; and
X5 = P or R; and

YCX1X2X3X4X5X6X7X8X9X10VF (6)

wherein, X1 = Q, A, or S;
X2 = S or A;
X3 = Y or W;
X4 = D or A;
X5 = S, D, or G;
X6 = S, N, or T;
X7 = S, L, N, or K;
X8 = V, S, N, or G;
X9 = G, L, V, or absent; and
X10 = P or absent.

In one embodiment, one or more of the antibody and an antigen-binding fragment thereof of the present invention may include one or more of the 25 antibodies shown in Table 1, including CK1, CK2, CK3, CL4, CL5, CL6, CB201, CL8, CL9, CL10, SK11, SK12, SK13, SK14, SK15, SK16, SK17, SL18, CB301_H3L1_A10, CB301_H3L1_A12, CB301_H3L1_E6, CB301_H3L1_F4, CB301_H3L1_G11, CB301_OPALTL_B5, and CB301_OPALTL_E6.

**[Table 1]**

| Antibod y | Phage Type | Domain | | Amino Acid Sequence (CDR1, CDR2, AND CDR3 are sequentially bolded and underlined.) | SEQ ID NO: |
|---|---|---|---|---|---|
| CK1 | Kappa | Heavy chain variabl e region | Full seque nce | EVQLLESGGGLVQPGGSLRLSCAASG FTFSRYAMTWVRQAPGKGLEWVSSM SGTGGTTYYADSVKGRFTISRDNSKNT LYLQMNSLRAEDTAVYYCARGAYGFD HWGQGTLVTVSS | 303 |
| | | | CDR1 | **FTFSRYAMTWVR** | 7 |
| | | | CDR2 | **SMSGTGGTTYYAD** | 8 |
| | | | CDR3 | **YCARGAYGFDHW** | 9 |
| | | Light chain | Full seque | EIVLTQSPGTLSLSPGERATLSCRASQ SIGNYLNWYQQKPGQAPRLLIYDASNL | 304 |
| | | variabl e region | nce | ETGIPDRFSGSGSGTDFTLTISRLEPED FAVYYCQQSSAIPYTFGQGTKVEIK | |
| | | | CDR1 | **CRASQSIGNYLNW** | 10 |
| | | | CDR2 | **DASNLETGIP** | 11 |
| | | | CDR3 | **YCQQSSAIPYTF** | 12 |
| CK2 | Kappa | Heavy chain variabl e region | Full seque nce | EVQLLESGGGLVQPGGSLRLSCAASG FTFSSYGMHWVRQAPGKGLEWVSAIS GSGTSIYYADSVKGRFTISRDNSKNTL YLQMNSLRAEDTAVYYCARGGTAFDY WGQGTLVTVSS | 307 |
| | | | CDR1 | **FTFSSYGMHWVR** | 19 |
| | | | CDR2 | **AISGSGTSIYYAD** | 20 |
| | | | CDR3 | **YCARGGTAFDYW** | 21 |
| | | Light chain variabl e region | Full seque nce | EIVLTQSPGTLSLSPGERATLSCRASQ RSDNYLAWYQQKPGQAPRLLIYDASN RATGIPDRFSGSGSGTDFTLTISRLEPE DFAVYYCQQSYSTPFTFGQGTKVETK | 308 |
| | | | CDR1 | **CRASQRSDNYLAW** | 22 |
| | | | CDR2 | **DASNRATGIP** | 23 |
| | | | CDR3 | **YCQQSYSTPFTF** | 24 |
| CK3 | Kappa | Heavy chain variabl e region | Full seque nce | RVQLLESGGGLVQPGGSLRLSCAASG FTFSSYAISWVRQAPGKGLEWVSATS GSGRATYYADSVKGRFTISRDNSKNTL YLQMNSLRAEDTAVYYCARDTWWEG YFDLWGQGTLVTVSS | 311 |
| | | | CDR1 | **FTFSSYAISWVR** | 31 |
| | | | CDR2 | **ATSGSGRATYYAD** | 32 |
| | | | CDR3 | **YCARDTWWEGYFDLW** | 33 |
| | | Light chain variabl e region | Full seque nce | EIVLTQSPGTLSLSPGERATLSCQASHI STHLNWYQQKPGQAPRLLIYGASSRA TGIPDRFSGSGSGTDFTLTISRLEPEDF AVYYCQQYNTYPPTFGQGTKVEIK | 312 |
| | | | CDR1 | **CQASHISTHLNW** | 34 |
| | | | CDR2 | **GASSRATGIP** | 35 |
| | | | CDR3 | **YCQQYNTYPPTF** | 36 |
| CL4 | Lambda | Heavy chain variabl e region | Full seque nce | RVQLLESGGGLVQPGGSLRLSCAASG FTFGSNYMSWVRQAPGKGLEWVSTIS GSGTSTYYADSLKGRFTISRDNSKNTL YLQMNSLRAEDTAVYYCARGMWGMD VWGQGTLVTVSS | 315 |
| | | | CDR1 | **FTFGSNYMSWVR** | 43 |
| | | | CDR2 | **TISGSGTSTYYAD** | 44 |
| | | | CDR3 | **YCARGMWGMDVW** | 45 |
| | | Light chain variabl e region | Full seque nce | QSVLTQPPSASGTPGQRVTISCTGKHR HTVNWYQLLPGTAPKLLIYLDSERPSG VPDRFSGSKSGTSASLAISGLRSEDEA DYYCQSYDSSSVVFGGGTKLTVL | 316 |
| | | | CDR1 | **CTGKHRHTVNW** | 46 |
| | | | CDR2 | **LDSERPSGVP** | 47 |
| | | | CDR3 | **YCQSYDSSSVVF** | 48 |
| CL5 | Lambda | Heavy chain variabl e region | Full seque nce | RVQLLESGGGLVQPGGSLRLSCAASG FTFSSYAMHWVRQAPGKGLEWVSSIS GGGYGTYYADSVKGRFTISRDNSKNTL YLQMNSLRAEDTAVYYCARSTVWAFDI WGQGTLVTVSS | 319 |
| | | | CDR1 | **FTFSSYAMHWVR** | 55 |
| | | | CDR2 | **SISGGGYGTYYAD** | 56 |
| | | | CDR3 | **YCARSTVWAFDIW** | 57 |
| | | Light chain variabl e region | Full seque nce | QSVLTQPPSASGTPGQRVTISCSGNNI GSKSVHWYQQLPGTAPKLLIYDVSKRP SERPDRFSGSKSGTSASLAISDLRSED EADYYCQSFDSSGTWIFGGGTKLTVL | 320 |
| | | | CDR1 | **CSGNNIGSKSVHW** | 58 |
| | | | CDR2 | **DVSKRPSERPD** | 59 |
| | | | CDR3 | **YCQSFDSSGTWIF** | 60 |
| CL6 | VH3VL1 | Heavy chain variabl e region | Full seque nce | EVQLLESGGGLVQPGGSLRLSCAASG FTFSSYGMHWVRQAPGKGLEWVSAIS GSGGSTYYADSVKGRFTISRDNSKNTL YLQMNSLRAEDTAVYYCAVSGAGRGF FDYWGQGTLVTVSS | 323 |
| | | | CDR1 | **FTFSSYGMHWVR** | 67 |
| | | | CDR2 | **AISGSGGSTYYAD** | 68 |
| | | | CDR3 | **YCAVSGAGRGFFDYW** | 69 |
| | | Light chain variabl e region | Full seque nce | QSVLTQPPSASGTPGQRVTISCSGSSS NIGSNYVYWYQQLPGTAPKLLIYEDNK RPSGVPDRFSGSKSGTSASLAISGLRS EDEADYYCSSYTSSSTVIFGGGTKLTV L | 324 |
| | | | CDR1 | **CSGSSSNIGSNYVYW** | 70 |
| | | | CDR2 | **EDNKRPSGVP** | 71 |
| | | | CDR3 | **YCSSYTSSSTVIF** | 72 |
| CB201 | VH3VL1 | Heavy chain variabl | Full seque nce | EVQLLESGGGLVQPGGSLRLSCAASG FTFSRYAMSWVRQAPGKGLEWVSAIS GSGGSTYYADSVKGRFTISRDNSKNTL | 327 |
| | | e region | | YLQMNSLRAEDTAVYYCARSSQGIFDI WGQGTLVTVSS | |
| | | | CDR1 | **FTFSRYAMSWVR** | 79 |
| | | | CDR2 | **AISGSGGSTYYAD** | 80 |
| | | | CDR3 | **YCARSSQGIFDIW** | 81 |
| | | Light chain variabl e region | Full seque nce | QSVLTQPPSASGTPGQRVTISCSGNNI GTRRVHWYQQLPDTAPKLLIYSKNNRP SGVPDRFSGSKSGTSASLAISGLRSED EADYYCAAWDDSLSGPVFGGGTKLTV L | 328 |
| | | | CDR1 | **CSGNNIGTRRVHW** | 82 |
| | | | CDR2 | **SKNNRPSGVP** | 83 |
| | | | CDR3 | **YCAAWDDSLSGPVF** | 84 |
| CL8 | VH3VL1 | Heavy chain variabl e region | Full seque nce | EVQLLESGGGLVQPGGSLRLSCAASG FTFSSYAMSWVRQAPGKGLEWVSAIS GSGGSTYYADSVKGRFTISRNNSKNTL YLQMNSLRAEDTAVYYCARSGRYADL TSGGQGTLVTVSS | 331 |
| | | | CDR1 | **FTFSSYAMSWVR** | 91 |
| | | | CDR2 | **AISGSGGSTYYAD** | 92 |
| | | | CDR3 | **YCARSGRYADLTSG** | 93 |
| | | Light chain variabl e region | Full seque nce | QSVLTQPPSASGTPGQRVTISCSGSNS NIGNNYVSWYQQLPDTPPKLLIYDNNK RPSGVPDRFSGSKSGTSASLAISGLRS EDEADYYCSSYTSSSTVMFGGGTKLT VL | 332 |
| | | | CDR1 | **CSGSNSNIGNNYVSW** | 94 |
| | | | CDR2 | **DNNKRPSGVP** | 95 |
| | | | CDR3 | **YCSSYTSSSTVMF** | 96 |
| CL9 | VH3VL1 | Heavy chain variabl e region | Full seque nce | EVQLLESGGGLVQPGGSLRLSCAASG FTFSSYYWSWVRQAPGKGLEWVSAIS GSGGSTYYADSVKGRFTISRDNSKNTL YLQMNSLRAEDTAVYYCARIDVYGFDI WGQGTLVTVSS | 335 |
| | | | CDR1 | **FTFSSYYWSWVR** | 103 |
| | | | CDR2 | **AISGSGGSTYYAD** | 104 |
| | | | CDR3 | **YCARIDVYGFDIW** | 105 |
| | | Light chain variabl e region | Full seque nce | QSVLTQPPSASGTPGQRVTISCSGSTS NIGTNYVYWYQQLPGTAPKLLIYDNNN RPSGVPDRFSGSKSGTSASLAISGLRS EDEADYYCQTWDSSTDVVFGGGTKLT VL | 336 |
| | | | CDR1 | **CSGSTSNIGTNYVYW** | 106 |
| | | | CDR2 | **DNNNRPSGVP** | 107 |
| | | | CDR3 | **YCQTWDSSTDVVF** | 108 |
| CL10 | VH3VL1 | Heavy chain variabl e region | Full seque nce | EVQLLESGGGLVQPGGSLRLSCAASG FTFSSYGMHWVRQAPGKGLEWVSAIS GSGGSTYYADSVKGRFTISRDNSKNTL YLQMNSLRAEDTAVYYCASGYGLMDV WGQGTLVTVSS | 339 |
| | | | CDR1 | **FTFSSYGMHWVR** | 115 |
| | | | CDR2 | **AISGSGGSTYYAD** | 116 |
| | | | CDR3 | **YCASGYGLMDVW** | 117 |
| | | Light chain variabl | Full seque nce | QSVLTQPPSASGTPGQRVTISCTRSSG IIASNYVQWYQQLPGTAPKLLIYRNNQ RPSGVPDRFSGSKSGTSASLAISGLRS | 340 |
| | | e region | | EDEADYYCSSYAGNNNLVFGGGTKLT VL | |
| | | | CDR1 | **CTRSSGIIASNYVQW** | 118 |
| | | | CDR2 | **RNNQRPSGVP** | 119 |
| | | | CDR3 | **YCSSYAGNNNLVF** | 120 |
| SK11 | Kappa | Heavy chain variabl e region | Full seque nce | RVQLLESGGGLVQPGGSLRLSCAASG FTFSTYGMHWVRQAPGKGLEWVSAIS GSGGSTYYADSVKGRFTISRDNSKNTL YLQMNSLRAEDTAVYYCARGLSGLDY WGQGTLVTVSS | 343 |
| | | | CDR1 | **FTFSTYGMHWVR** | 127 |
| | | | CDR2 | **AISGSGGSTYYAD** | 128 |
| | | | CDR3 | **YCARGLSGLDYW** | 129 |
| | | Light chain variabl e region | Full seque nce | EIVLTQSPGTLSLSPGERATLSCRSSQ GITNYLAWYQQKPGQAPRLLIYDASNR ATGIPDRFSGSGSGTDFTLTISRLEPED FAVYYCQQSYSTPLTFGQGTKVEIK | 344 |
| | | | CDR1 | **CRSSQGITNYLAW** | 130 |
| | | | CDR2 | **DASNRATGIP** | 131 |
| | | | CDR3 | **YCQQSYSTPLTF** | 132 |
| SK12 | Kappa | Heavy chain variabl e region | Full seque nce | RVQLLESGGGLVQPGGSLRLSCAASG FTFSSYAMHWVRQAPGKGLEWVSAIS GSGGDTYHADSVKGRFTISRDNSKNTL YLQMNSLRAEDTAVYYCTRGLSGFDY WGQGTLVTVSS | 347 |
| | | | CDR1 | **FTFSSYAMHWVR** | 139 |
| | | | CDR2 | **AISGSGGDTYHAD** | 140 |
| | | | CDR3 | **YCTRGLSGFDYW** | 141 |
| | | Light chain variabl e region | Full seque nce | EIVLTQSPGTLSLSPGERATLSCRASQ SISSYLNWYQQKPGQAPRLLIYDASNR APGIPDRFSGSGSGTDFTLTISRLEPED FAVYYCQQSYSIPITFGQGTKVEIKFSD | 348 |
| | | | CDR1 | **CRASQSISSYLNW** | 142 |
| | | | CDR2 | **DASNRAPGIP** | 143 |
| | | | CDR3 | **YCQQSYSIPITF** | 144 |
| SK13 | Kappa | Heavy chain variabl e region | Full seque nce | RVQLLESGGGLVQPGGSLRLSCAASG FTFSDYAMSWVRQAPGKGLEWVSSIS SSSSYIYYTDSVKGRFTISRDNSKNTLY LQMNSLRAEDTAVYYCARGGYGFDY WGQGTLVTVSS | 351 |
| | | | CDR1 | **FTFSDYAMSWVR** | 151 |
| | | | CDR2 | **SISSSSSYIYYTD** | 152 |
| | | | CDR3 | **YCARGGYGFDYW** | 153 |
| | | Light chain variabl e region | Full seque nce | EIVLTQSPGTLSLSPGERATLSCRASQ SISSYLNWYQQKPGQAPRLLIYSASSR PQGIPDRFSGSGSGTDFTLTISRLEPED FAVYYCQQYDDLPFTFGQGTKVEIK | 352 |
| | | | CDR1 | **CRASQSISSYLNW** | 154 |
| | | | CDR2 | **SASSRPQGIP** | 155 |
| | | | CDR3 | **YCQQYDDLPFTF** | 156 |
| SK14 | Kappa | Heavy chain variabl e region | Full seque nce | EVQLLESGGGLVQPGGSLRLSCAASG FTFSNFAIAWVRQAPGKGLEWVSAISG RGTSTYYADSVKGRFTISRDNSKNTLY LQMNSLRAEDTAVYYCARGVSGFDSW GQGTLVTVSS | 355 |
| | | | CDR1 | **FTFSNFAIAWVR** | 163 |
| | | | CDR2 | **AISGRGTSTYYAD** | 164 |
| | | | CDR3 | **YCARGVSGFDSW** | 165 |
| | | Light chain variabl e region | Full seque nce | EIVLTQSPGTLSLSPGERATLSCRASQ SISSHLAWYQQKPGQAPRLLIYDTSNR ATGIPDRFSGSGSGTDFTLTISRLEPED FAVYYCQQSYSTPFTFGQGTKVEIK | 356 |
| | | | CDR1 | **CRASQSISSHLAW** | 166 |
| | | | CDR2 | **DTSNRATGIP** | 167 |
| | | | CDR3 | **YCQQSYSTPFTF** | 168 |
| SK15 | Kappa | Heavy chain variabl e region | Full seque nce | RVQLLESGGGLVQPGGSLRLSCAASG FTFSSYAMHWVRQAPGKGLEWVSAIN GSGGSTYYADSVKGRFTISRDNSKNTL YLQTNSLRAEDTAVYYCARGLQGFDY WGQGTLVTVSS | 359 |
| | | | CDR1 | **FTFSSYAMHWVR** | 175 |
| | | | CDR2 | **AINGSGGSTYYAD** | 176 |
| | | | CDR3 | **YCARGLQGFDYW** | 177 |
| | | Light chain variabl e region | Full seque nce | EIVLTQSPGTLSLSPGERATLSCQASQ DITNYLNWYQQKPGQAPRLLIYDASSL ETGIPDRFSGSGSGTDFTLTISRLEPED FAVYYCQQSYSTPITFGQGTKVEIK | 360 |
| | | | CDR1 | **CQASQDITNYLNW** | 178 |
| | | | CDR2 | **DASSLETGIP** | 179 |
| | | | CDR3 | **YCQQSYSTPITF** | 180 |
| SK16 | Kappa | Heavy chain variabl e | Full seque nce | RVQLLESGGGLVQPGGSLRLSCAASG FTFSSYAMSWVRQAPGKGLEWVSAIN GSGGSTLYADSVKGRFTISRDNSKNTL YLQMNSLRAEDTAVYYCARGVSGFDS | 363 |
| | | region | | **W**GQGTLVTVSS | |
| | | | CDR1 | **FTFSSYAMSWVR** | 187 |
| | | | CDR2 | **AINGSGGSTLYAD** | 188 |
| | | | CDR3 | **YCARGVSGFDSW** | 189 |
| | | Light chain variabl e region | Full seque nce | EIVLTQSPGTLSLSPGERATLSCRISQSI SSYLNWYQQKPGQAPRLLIYDASLRA TGIPDRFSGSGSGTDFTLTISRLEPEDF AVYYCQQSYKTPITFGQGTKVEIK | 364 |
| | | | CDR1 | **CRISQSISSYLNW** | 190 |
| | | | CDR2 | **DASLRATGIP** | 191 |
| | | | CDR3 | **YYCQQSYKTPITF** | 192 |
| SK17 | Kappa | Heavy chain variabl e region | Full seque nce | EVQLLESGGGLVQPGGSLRLSCAASG FTFSSYYWSWVRQAPGKGLEWVSTIT GSGGSTDYANSVKGRFTISRDNSKNTL YLQMNSLRAEDTAVYYCATGGGIFDY WGQGTLVTVSS | 367 |
| | | | CDR1 | **FTFSSYYWSWVR** | 199 |
| | | | CDR2 | **TITGSGGSTDYAN** | 200 |
| | | | CDR3 | **YCATGGGIFDYW** | 201 |
| | | Light chain variabl e region | Full seque nce | EIVLTQSPGTLSLSPGERATLSCQASQ TISNYLNWYQQKPGQAPRLLIYDASNR ATGIPDRFSGSGSGTDFTLTISRLEPED FAVYYCQQYNSYPPSFGQGTKVEIK | 368 |
| | | | CDR1 | **CQASQTISNYLNW** | 202 |
| | | | CDR2 | **DASNRATGIP** | 203 |
| | | | CDR3 | **YCQQYNSYPPSF** | 204 |
| SL18 | Lambda | Heavy chain | Full seque | RVQLLESGGGLVQPGGSLRLSCAASG FTFSDYHMHWVRQAPGKGLEWVSTIS | 371 |
| | | variabl e region | nce | SSGGYTYYAESVKSRFTISRDNSKNTL YLQMNSLRAEDTAVYYCARSIRLPLDY WGQGTLVTVSS | |
| | | | CDR1 | **FTFSDYHMHWVR** | 211 |
| | | | CDR2 | **TISSSGGYTYYAE** | 212 |
| | | | CDR3 | **YCARSIRLPLDYW** | 213 |
| | | Light chain variabl e region | Full seque nce | QSVLTQPPSASGTPGQRVTISCSGNNI GSKGVHWYQQLPGTAPKLLIYEDSKR PSGVRDRFSGSKSGTSASLAISGLRSE DEADYYCQSYDSTKGVVFGGGTKLTV L | 372 |
| | | | CDR1 | **CSGNNIGSKGVHW** | 214 |
| | | | CDR2 | **EDSKRPSGVR** | 215 |
| | | | CDR3 | **YCQSYDSTKGVVF** | 216 |
| CB301_ H3L1_A 10 | VH3VL1 | Heavy chain variabl e region | Full seque nce | EVQLLESGGGLVQSGGSLRLSCAASG FTFSSYGMHWVRQAPGKGLEWVSAIS GSGGSTYYADSVKGRFTISRDNSKNTL YLQMNSLRAEDTAVYYCVRGYGAMDV WGQGTLVTVSS | 375 |
| | | | CDR1 | **FTFSSYGMHWVR** | 223 |
| | | | CDR2 | **AISGSGGSTYYAD** | 224 |
| | | | CDR3 | **YCVRGYGAMDVW** | 225 |
| | | Light chain variabl e region | Full seque nce | QSVLTQPPSASGTPGQRVTISCTRSSG SIASNYVQWYQQLPGTAPKLLIYRNNQ RPSGVPDRFSGSKSGTSASLAISGLRS EDEADYYCSSYTTSSTLVFGGGTKLTV L | 376 |
| | | | CDR1 | **CTRSSGSIASNYVQW** | 226 |
| | | | CDR2 | **RNNQRPSGVP** | 227 |
| | | | CDR3 | **YCSSYTTSSTLVF** | 228 |
| CB301_ H3L1_A 12 | VH3VL1 | Heavy chain variabl e region | Full seque nce | EVQLLESGGGLVQPGGSLRLSCAASG FTFSSYAMHWVRQAPGKGLEWVSAIS GSGGSTYYADSVKGRFTISRDNSKNTL YLQMNSLRAKDTAVYYCASGYGLMDV WGQGTLVTVSS | 379 |
| | | | CDR1 | **FTFSSYAMHWVR** | 235 |
| | | | CDR2 | **AISGSGGSTYYAD** | 236 |
| | | | CDR3 | **YCASGYGLMDVW** | 237 |
| | | Light chain variabl e region | Full seque nce | QSVLTQPPSASGTPGQRVTISCTGTSS DVGNYNLVSWYQQLPGTAPKLLIYSNN QRPSGVPDRFSGSKSGTSASLAISGLR SEDEADYYCSSYTGSNALLFGGGTKL TVL | 380 |
| | | | CDR1 | **CTGTSSDVGNYNLVSW** | 238 |
| | | | CDR2 | **SNNQRPSGVP** | 239 |
| | | | CDR3 | **YCSSYTGSNALLF** | 240 |
| CB301_ H3L1_E 6 | VH3VL1 | Heavy chain variabl e region | Full seque nce | EVQLLESGGGLVQPGGSLRLSCAASG FTFSSYAMSWVRQAPGKGLEWVSAIS GSGGSTYYADSVKGRFTISRDNSKNTL YLQMNSLRAEDTAVYYCARWHYSFDY WGQGTLVTVSS | 383 |
| | | | CDR1 | **FTFSSYAMSWVR** | 247 |
| | | | CDR2 | **AISGSGGSTYYAD** | 248 |
| | | | CDR3 | **YCARWHYSFDYW** | 249 |
| | | Light chain | Full seque | QSVLTQPPSASGTPGQRVTISCRGNNI GSKRVHWYQQLPGTAPKLLIYSYNHR | 384 |
| | | variabl e region | nce | PSGVPDRFSGSKSGTSASLAITGLRSE DEADYYCNTWDDSLEGPVFGGGTKLT VL | |
| | | | CDR1 | **CRGNNIGSKRVHW** | 250 |
| | | | CDR2 | **SYNHRPSGVP** | 251 |
| | | | CDR3 | **YCNTWDDSLEGPVF** | 252 |
| CB301_ H3L1_F 4 | VH3VL1 | Heavy chain variabl e region | Full seque nce | EVQLLESGGGLVQPGGSLRLSCAASG FTFSGYAMSWVRQAPGKGLEWVSAIS GSGGSTYYADSVKGRFTISRDNSKNTL YLQMNSLRAEDTAVYYCARSPSGLFD YWGQGTLVTVSS | 387 |
| | | | CDR1 | **FTFSGYAMSWVR** | 259 |
| | | | CDR2 | **AISGSGGSTYYAD** | 260 |
| | | | CDR3 | **YCARSPSGLFDYW** | 261 |
| | | Light chain variabl e region | Full seque nce | QSVLTQPPSASGTPGQRVTISCGGNNI GSKRVHWYQQLPGTAPKLLIYNTSNK HSGVPDRFSGSKSGTSASLAISGLRSE DEADYYCSSYLQQHSLFGGGTKLTVL | 388 |
| | | | CDR1 | **CGGNNIGSKRVHW** | 262 |
| | | | CDR2 | **NTSNKHSGVP** | 263 |
| | | | CDR3 | **YCSSYLQQHSLF** | 264 |
| CB301_ H3L1_G 11 | VH3VL1 | Heavy chain variabl e region | Full seque nce | EVQLLESGGGLVQPGGSSSASSCAAS GFTFSSYAMSWVRQAPGKGLEWVSAI SGSGGSTYYADSVKGRFTISRDNSKNT LYLQMNSLRAEDTAVYYCTRFVGAIGA FDYWGQGTLVTVSS | 391 |
| | | | CDR1 | **FTFSSYAMSWVR** | 271 |
| | | | CDR2 | **AISGSGGSTYYAD** | 272 |
| | | | CDR3 | **YCTRFVGAIGAFDYW** | 273 |
| | | Light chain variabl e region | Full seque nce | QSVLTQPPSASGTPGQRVTISCSGNNI GSRSVHWYQQLPGTAPKLLIYRNNQR PSGVPDRFSGSKSGTSASLAISGLRSE DEADYYCAAWDDSLSGPVFGGGTKLT VL | 392 |
| | | | CDR1 | **CSGNNIGSRSVHW** | 274 |
| | | | CDR2 | **RNNQRPSGVP** | 275 |
| | | | CDR3 | **YCAAWDDSLSGPVF** | 276 |
| CB301_ OPALT L_B5 | OPALTL | Heavy chain variabl e region | Full seque nce | EVQLLESGGGLVQPGGSLRLSCAASG FTFSHYAMSWVRQAPGKGLEWVSAIS GSGGSTYYADSVKGRFTISRDNSKNTL YLQMNSLRAEDTAVYYCARGWDSPTL TYFDSWGQGTLVTVSS | 395 |
| | | | CDR1 | **FTFSHYAMSWVR** | 283 |
| | | | CDR2 | **AISGSGGSTYYAD** | 284 |
| | | | CDR3 | **YCARGWDSPTLTYFDSW** | 285 |
| | | Light chain variabl e region | Full seque nce | QSVLTQPPSASGTPGQRVTISCSGTSS NIGNNDVSWYQQLPGTAPKLLIYQDTK RPSGVPDRFSGSKSGTSASLAISGLRS EDEADYYCAAWDDSLSGPVFGGGTKL TVL | 396 |
| | | | CDR1 | **CSGTSSNIGNNDVSW** | 286 |
| | | | CDR2 | **QDTKRPSGVP** | 287 |
| | | | CDR3 | **YCAAWDDSLSGPVF** | 288 |
| CB301_ OPALT L_E6 | OPALTL | Heavy chain variabl | Full seque nce | EVQLLESGGGLVQPGGSLRLSCAASG FTFSSYGMHWVRQAPGKGLEWVSAIS GSGGYTYYADSVKGRFTISRDNSKNTL | 399 |
| | | e region | | YLQMNSLRAEDTAVYYCARWHYSFDY WGQGTLVTVSS | |
| | | | CDR1 | **FTFSSYGMHWVR** | 295 |
| | | | CDR2 | **AISGSGGYTYYAD** | 296 |
| | | | CDR3 | **YCARWHYSFDYW** | 297 |
| | | Light chain variabl e region | Full seque nce | QSVLTQPPSASGTPGQRVTISCSGSSS NIGNNYVSWYQQLPGTAPKLLIYRNNQ RPSGVPDRFSGSKSGTSASLAISGLRS EDEADYYCQSYDNSNVLFGGGTKLTV L | 400 |
| | | | CDR1 | **CSGSSSNIGNNYVSW** | 298 |
| | | | CDR2 | **RNNQRPSGVP** | 299 |
| | | | CDR3 | **YCQSYDNSNVLF** | 300 |

In the present invention, the CDRs of the variable region were determined by a conventional method according to the system devised by Kabat *et al.* (see [Kabat et al., Sequences of Proteins of Immunological Interest (5th), National Institutes of Health, Bethesda, MD. (1991)]). Although the CDR numbering used in the present invention follows the Kabat method, antibodies including CDRs determined by other methods such as the IMGT method, Chothia method, and AbM method are also included within the scope of the present invention.

If the antibody of the present invention includes a constant region, the antibody may include a constant region derived from IgG, IgA, IgD, IgE, IgM, or a combination or hybrid thereof.

In the present invention, the term "combination" means that when forming a dimer or multimer, a polypeptide encoding a single-chain immunoglobulin constant region of the same origin forms a combination with a single-chain polypeptide of a different origin. For example, a dimer or multimer may be formed from two or more constant regions selected from the group consisting of the constant regions of IgG, IgA, IgD, IgE, and IgM.

In the present invention, the term "hybrid" means that sequences corresponding to immunoglobulin heavy chain constant regions of two or more different origins are present within a single-chain immunoglobulin heavy chain constant region. For example, a hybrid of domains consisting of one to four domains selected from the group consisting of CH1, CH2, CH3, and CH4 of IgG, IgA, IgD, IgE, and IgM is possible.

Additionally, in the present invention, the origins of a variable region and a constant region of an antibody may be the same or different, and the origins of the CDRs, the variable region excluding the CDRs, and the constant region may be the same or different.

In one embodiment of the previously described embodiments, one or more of the anti-CD300c antibody and an antigen-binding fragment thereof may preferably include a sequence having sequence identity of 80% or more, 85% or more, 90% or more, more preferably 95% or more, and most preferably 98% or more compared to the CDR sequences or heavy chain variable region and light chain variable region sequences presented in Table 1.

In one embodiment, variants of the amino acid sequence of the antibody of the present invention may be considered. The variants prepared at a level generally practiced in the art to maintain or enhance the function of the anti-CD300c antibody and an antigen-binding fragment thereof of the present invention may also be included in the present invention as long as these variants of the anti-CD300c antibody and an antigen-binding fragment thereof of the present invention have the same or corresponding characteristics as the present invention. For example, these variants may be those which improve the binding affinity and/or other biological properties of the antibody. An amino acid sequence variant of the antibody may be prepared by introducing appropriate modification into the nucleotide sequence encoding the molecule or by peptide synthesis. Such modification includes, for example, deletions of residues from the amino acid sequence of the antibody, and/or insertions of residues into such amino acid sequence, and/or substitutions of residues within such amino acid sequence. Any combination of deletions, insertions, and substitutions may be performed to reach the final construct, but the final construct should retain the desired properties, such as an antigen-binding property. Sites of interest for inducing substitution mutations include the heavy chain variable regions (HVRs) and framework regions (FRs). Conservative substitutions are provided under the item "preferred substitutions" in Table 2 and are further described below in relation to amino acid side chain classes (1) to (6). Amino acid substitutions may be introduced into a molecule of interest and product screened for desired activities, such as retained/improved antigen binding, reduced immunogenicity, or improved ADCC or CDC.

**[Table 2]**

| Original Residue | Exemplary Substitution | Preferred Substitution |
|---|---|---|
| Ala (A) | Val; Leu; Ile | Val |
| Arg (R) | Lys; Gln; Asn | Lys |
| Asn (N) | Gln; His; Asp; Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Ile |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to their conventional side chain properties as follows:
(1) Hydrophobic: Norleucine, Met, Ala, Val, Leu, Ile;
(2) Neutral hydrophilic: Cys, Ser, Thr, Asn, Gln;
(3) Acidic: Asp, Glu;
(4) Basic: His, Lys, Arg;
(5) Residues affecting the chain orientation: Gly, Pro; and
(6) Aromatic: Trp, Tyr, Phe.

Non-conservative substitutions involve exchanging a constituent of one of these classes with a constituent of another class.

In the present invention, the term "amino acid sequence variant" includes substantial variants in which amino acid substitutions are present in one or more residues in the hypervariable region of the parent antibody binding molecule (*e.g.,* a humanized or human antibody). Generally, variants selected and prepared for further study will have modifications of specific biological properties, such as improvements (*e.g.,* increased affinity, reduced immunogenicity), compared to the parent antibody binding molecule or will substantially retain specific biological properties of the parent antigen-binding molecule. An exemplary substitution variant is an affinity-matured antibody, which can, for example, be conveniently produced using phage display-based affinity maturation techniques known in the art. Briefly, one or more residues in the hypervariable region (HVR) are mutated, and the variant antigen-binding molecules are displayed on phages and screened for specific biological activity (*e.g.,* binding affinity). In certain embodiments, substitutions, insertions, or deletions may occur within one or more HVRs as long as such modifications do not substantially reduce the ability of the antigen-binding molecule to bind to an antigen. For example, conservative modifications that do not substantially reduce binding affinity (*e.g.,* conservative substitutions as provided herein) may be made in the HVR. Amino acid sequence insertions may include not only the insertions within the sequence of single or multiple amino acid residues, but also amino-terminal and/or carboxy-terminal fusions ranging from a single residue to a polypeptide of over a hundred residues in length. Examples of terminal insertions include antibodies with an N-terminal methionyl residue. Other insertion variants of the molecule may include fusions to the N-terminus or C-terminus of a polypeptide that increases the serum half-life of the antibody. Additionally, other insertion variants of the molecule may include fusions to the N-terminus or C-terminus of a polypeptide that facilitates passage through the blood-brain barrier (BBB).

In the present invention, the polynucleotide sequences encoding CDR1 to CDR3 of the heavy chain variable region of the antibody CK1 are shown in SEQ ID NO: 1 to SEQ ID NO: 3, respectively; the polynucleotide sequences encoding CDR1 to CDR3 of the light chain variable region of the antibody CK1 are shown in SEQ ID NO: 4 to SEQ ID NO: 6, respectively; the amino acid sequences of CDR1 to CDR3 of the heavy chain variable region of the antibody CK1 are shown in SEQ ID NO: 7 to SEQ ID NO: 9, respectively; and the amino acid sequences of CDR1 to CDR3 of the light chain variable region of the antibody CK1 are shown in SEQ ID NO: 10 to SEQ ID NO: 12, respectively.

The polynucleotide sequences encoding CDR1 to CDR3 of the heavy chain variable region of the antibody CK2 are shown in SEQ ID NO: 13 to SEQ ID NO: 15, respectively; the polynucleotide sequences encoding CDR1 to CDR3 of the light chain variable region of the antibody CK2 are shown in SEQ ID NO: 16 to SEQ ID NO: 18, respectively; the amino acid sequences of CDR1 to CDR3 of the heavy chain variable region of the antibody CK2 are shown in SEQ ID NO: 19 to SEQ ID NO: 21, respectively; and the amino acid sequences of CDR1 to CDR3 of the light chain variable region of the antibody CK2 are shown in SEQ ID NO: 22 to SEQ ID NO: 24, respectively.

The polynucleotide sequences encoding CDR1 to CDR3 of the heavy chain variable region of the antibody CK3 are shown in SEQ ID NO: 25 to SEQ ID NO: 27, respectively; the polynucleotide sequences encoding CDR1 to CDR3 of the light chain variable region of the antibody CK3 are shown in SEQ ID NO: 28 to SEQ ID NO: 30, respectively; the amino acid sequences of CDR1 to CDR3 of the heavy chain variable region of the antibody CK3 are shown in SEQ ID NO: 31 to SEQ ID NO: 33, respectively; and the amino acid sequences of CDR1 to CDR3 of the light chain variable region of the antibody CK3 are shown in SEQ ID NO: 34 to SEQ ID NO: 36, respectively.

The polynucleotide sequences encoding CDR1 to CDR3 of the heavy chain variable region of the antibody CL4 are shown in SEQ ID NO: 37 to SEQ ID NO: 39, respectively; the polynucleotide sequences encoding CDR1 to CDR3 of the light chain variable region of the antibody CL4 are shown in SEQ ID NO: 40 to SEQ ID NO: 42, respectively; the amino acid sequences of CDR1 to CDR3 of the heavy chain variable region of the antibody CL4 are shown in SEQ ID NO: 43 to SEQ ID NO: 45, respectively; and the amino acid sequences of CDR1 to CDR3 of the light chain variable region of the antibody CL4 are shown in SEQ ID NO: 46 to SEQ ID NO: 48, respectively.

The polynucleotide sequences encoding CDR1 to CDR3 of the heavy chain variable region of the antibody CL5 are shown in SEQ ID NO: 49 to SEQ ID NO: 51, respectively; the polynucleotide sequences encoding CDR1 to CDR3 of the light chain variable region of the antibody CL5 are shown in SEQ ID NO: 52 to SEQ ID NO: 54, respectively; the amino acid sequences of t CDR1 to CDR3 of the heavy chain variable region of the antibody CL5 are shown in SEQ ID NO: 55 to SEQ ID NO: 57, respectively; and the amino acid sequences of CDR1 to CDR3 of the light chain variable region of the antibody CL5 are shown in SEQ ID NO: 58 to SEQ ID NO: 60, respectively.

The polynucleotide sequences encoding CDR1 to CDR3 of the heavy chain variable region of the antibody CL6 are shown in SEQ ID NO: 61 to SEQ ID NO: 63, respectively; the polynucleotide sequences encoding CDR1 to CDR3 of the light chain variable region of the antibody CL6 are shown in SEQ ID NO: 64 to SEQ ID NO: 66, respectively; the amino acid sequences of CDR1 to CDR3 of the heavy chain variable region of the antibody CL6 are shown in SEQ ID NO: 67 to SEQ ID NO: 69, respectively; and the amino acid sequences of CDR1 to CDR3 of the light chain variable region of the antibody CL6 are shown in SEQ ID NO: 70 to SEQ ID NO: 72, respectively.

The polynucleotide sequences encoding CDR1 to CDR3 of the heavy chain variable region of the antibody CB201 are shown in SEQ ID NO: 73 to SEQ ID NO: 75, respectively; the polynucleotide sequences encoding CDR1 to CDR3 of the light chain variable region of the antibody CB201 are shown in SEQ ID NO: 76 to SEQ ID NO: 78, respectively; the amino acid sequences of CDR1 to CDR3 of the heavy chain variable region of the antibody CB201 are shown in SEQ ID NO: 79 to SEQ ID NO: 81, respectively; and the amino acid sequences of CDR1 to CDR3 of the light chain variable region of the antibody CB201 are shown in SEQ ID NO: 82 to SEQ ID NO: 84, respectively.

The polynucleotide sequences encoding CDR1 to CDR3 of the heavy chain variable region of the antibody CL8 are shown in SEQ ID NO: 85 to SEQ ID NO: 87, respectively; the polynucleotide sequences encoding CDR1 to CDR3 of the light chain variable region of the antibody CL8 are shown in SEQ ID NO: 88 to SEQ ID NO: 90, respectively; the amino acid sequences of CDR1 to CDR3 of the heavy chain variable region of the antibody CL8 are shown in SEQ ID NO: 91 to SEQ ID NO: 93, respectively; and the amino acid sequences of CDR1 to CDR3 of the light chain variable region of the antibody CL8 are shown in SEQ ID NO: 94 to SEQ ID NO: 96, respectively.

The polynucleotide sequences encoding CDR1 to CDR3 of the heavy chain variable region of the antibody CL9 are shown in SEQ ID NO: 97 to SEQ ID NO: 99, respectively; the polynucleotide sequences encoding CDR1 to CDR3 of the light chain variable region of the antibody CL9 are shown in SEQ ID NO: 100 to SEQ ID NO: 102, respectively; the amino acid sequences of CDR1 to CDR3 of the heavy chain variable region of the antibody CL9 are shown in SEQ ID NO: 103 to SEQ ID NO: 105, respectively; and the amino acid sequences of CDR1 to CDR3 of the light chain variable region of the antibody CL9 are shown in SEQ ID NO: 106 to SEQ ID NO: 108, respectively.

The polynucleotide sequences encoding CDR1 to CDR3 of the heavy chain variable region of the antibody CL10 are shown in SEQ ID NO: 109 to SEQ ID NO: 111, respectively; the polynucleotide sequences encoding CDR1 to CDR3 of the light chain variable region of the antibody CL10 are shown in SEQ ID NO: 112 to SEQ ID NO: 114, respectively; the amino acid sequences of CDR1 to CDR3 of the heavy chain variable region of the antibody CL10 are shown in SEQ ID NO: 115 to SEQ ID NO: 117, respectively; and the amino acid sequences of CDR1 to CDR3 of the light chain variable region of the antibody CL10 are shown in SEQ ID NO: 118 to SEQ ID NO: 120, respectively.

The polynucleotide sequences encoding CDR1 to CDR3 of the heavy chain variable region of the antibody SK11 are shown in SEQ ID NO: 121 to SEQ ID NO: 123, respectively; the polynucleotide sequences encoding CDR1 to CDR3 of the light chain variable region of the antibody SK11 are shown in SEQ ID NO: 124 to SEQ ID NO: 126, respectively; the amino acid sequences of CDR1 to CDR3 of the heavy chain variable region of the antibody SK11 are shown in SEQ ID NO: 127 to SEQ ID NO: 129, respectively; and the amino acid sequences of CDR1 to CDR3 of the light chain variable region of the antibody SK11 are shown in SEQ ID NO: 130 to SEQ ID NO: 132, respectively.

The polynucleotide sequences encoding CDR1 to CDR3 of the heavy chain variable region of the antibody SK12 are shown in SEQ ID NO: 133 to SEQ ID NO: 135, respectively; the polynucleotide sequences encoding CDR1 to CDR3 of the light chain variable region of the antibody SK12 are shown in SEQ ID NO: 136 to SEQ ID NO: 138, respectively; the amino acid sequences of CDR1 to CDR3 of the heavy chain variable region of the antibody SK12 are shown in SEQ ID NO: 139 to SEQ ID NO: 141, respectively; and the amino acid sequences of CDR1 to CDR3 of the light chain variable region of the antibody SK12 are shown in SEQ ID NO: 142 to SEQ ID NO: 144, respectively.

The polynucleotide sequences encoding CDR1 to CDR3 of the heavy chain variable region of the antibody SK13 are shown in SEQ ID NO: 145 to SEQ ID NO: 147, respectively; the polynucleotide sequences encoding CDR1 to CDR3 of the light chain variable region of the antibody SK13 are shown in SEQ ID NO: 148 to SEQ ID NO: 150, respectively; the amino acid sequences of CDR1 to CDR3 of the heavy chain variable region of the antibody SK13 are shown in SEQ ID NO: 151 to SEQ ID NO: 153, respectively; and the amino acid sequences of CDR1 to CDR3 of the light chain variable region of the antibody SK13 are shown in SEQ ID NO: 154 to SEQ ID NO: 156, respectively.

The polynucleotide sequences encoding CDR1 to CDR3 of the heavy chain variable region of the antibody SK14 are shown in SEQ ID NO: 157 to SEQ ID NO: 159, respectively; the polynucleotide sequences encoding CDR1 to CDR3 of the light chain variable region of the antibody SK14 are shown in SEQ ID NO: 160 to SEQ ID NO: 162, respectively; the amino acid sequences of CDR1 to CDR3 of the heavy chain variable region of the antibody SK14 are shown in SEQ ID NO: 163 to SEQ ID NO: 165, respectively; and the amino acid sequences of CDR1 to CDR3 of the light chain variable region of the antibody SK14 are shown in SEQ ID NO: 166 to SEQ ID NO: 168, respectively.

The polynucleotide sequences encoding CDR1 to CDR3 of the heavy chain variable region of the antibody SK15 are shown in SEQ ID NO: 169 to SEQ ID NO: 171, respectively; the polynucleotide sequences encoding CDR1 to CDR3 of the light chain variable region of the antibody SK15 are shown in SEQ ID NO: 172 to SEQ ID NO: 174, respectively; the amino acid sequences of CDR1 to CDR3 of the heavy chain variable region of the antibody SK15 are shown in SEQ ID NO: 175 to SEQ ID NO: 177, respectively; and the amino acid sequences of CDR1 to CDR3 of the light chain variable region of the antibody SK15 are shown in SEQ ID NO: 178 to SEQ ID NO: 180, respectively.

The polynucleotide sequences encoding CDR1 to CDR3 of the heavy chain variable region of the antibody SK16 are shown in SEQ ID NO: 181 to SEQ ID NO: 183, respectively; the polynucleotide sequences encoding CDR1 to CDR3 of the light chain variable region of the antibody SK16 are shown in SEQ ID NO: 184 to SEQ ID NO: 186, respectively; the amino acid sequences of CDR1 to CDR3 of the heavy chain variable region of the antibody SK16 are shown in SEQ ID NO: 187 to SEQ ID NO: 189, respectively; and the amino acid sequences of CDR1 to CDR3 of the light chain variable region of the antibody SK16 are shown in SEQ ID NO: 190 to SEQ ID NO: 192, respectively.

The polynucleotide sequences encoding CDR1 to CDR3 of the heavy chain variable region of the antibody SK17 are shown in SEQ ID NO: 193 to SEQ ID NO: 195, respectively; the polynucleotide sequences encoding CDR1 to CDR3 of the light chain variable region of the antibody SK17 are shown in SEQ ID NO: 196 to SEQ ID NO: 198, respectively; the amino acid sequences of CDR1 to CDR3 of the heavy chain variable region of the antibody SK17 are shown in SEQ ID NO: 199 to SEQ ID NO: 201, respectively; and the amino acid sequences of CDR1 to CDR3 of the light chain variable region of the antibody SK17 are shown in SEQ ID NO: 202 to SEQ ID NO: 204, respectively.

The polynucleotide sequences encoding CDR1 to CDR3 of the heavy chain variable region of the antibody SL18 are shown in SEQ ID NO: 205 to SEQ ID NO: 207, respectively; the polynucleotide sequences encoding CDR1 to CDR3 of the light chain variable region of the antibody SL18 are shown in SEQ ID NO: 208 to SEQ ID NO: 210, respectively; the amino acid sequences of CDR1 to CDR3 of the heavy chain variable region of the antibody SL18 are shown in SEQ ID NO: 211 to SEQ ID NO: 213, respectively; and the amino acid sequences of CDR1 to CDR3 of the light chain variable region of the antibody SL18 are shown in SEQ ID NO: 214 to SEQ ID NO: 216, respectively.

The polynucleotide sequences encoding CDR1 to CDR3 of the heavy chain variable region of the antibody CB301_H3L1_A10 are shown in SEQ ID NO: 217 to SEQ ID NO: 219, respectively; the polynucleotide sequences encoding CDR1 to CDR3 of the light chain variable region of the antibody CB301_H3L1_A10 are shown in SEQ ID NO: 220 to SEQ ID NO: 222, respectively; the amino acid sequences of CDR1 to CDR3 of the heavy chain variable region of the antibody CB301_H3L1_A10 are shown in SEQ ID NO: 223 to SEQ ID NO: 225, respectively; and the amino acid sequences of CDR1 to CDR3 of the light chain variable region of the antibody CB301_H3L1_A10 are shown in SEQ ID NO: 226 to SEQ ID NO: 228, respectively.

The polynucleotide sequences encoding CDR1 to CDR3 of the heavy chain variable region of the antibody CB301_H3L1_A12 are shown in SEQ ID NO: 229 to SEQ ID NO: 231, respectively; the polynucleotide sequences encoding CDR1 to CDR3 of the light chain variable region of the antibody CB301_H3L1_A12 are shown in SEQ ID NO: 232 to SEQ ID NO: 234, respectively; the amino acid sequences of CDR1 to CDR3 of the heavy chain variable region of the antibody CB301_H3L1_A12 are shown in SEQ ID NO: 235 to SEQ ID NO: 237, respectively; and the amino acid sequences of CDR1 to CDR3 of the light chain variable region of the antibody CB301_H3L1_A12 are shown in SEQ ID NO: 238 to SEQ ID NO: 240, respectively.

The polynucleotide sequences encoding CDR1 to CDR3 of the heavy chain variable region of the antibody CB301_H3L1_E6 are shown in SEQ ID NO: 241 to SEQ ID NO: 243, respectively; the polynucleotide sequences CDR1 to CDR3 of encoding the light chain variable region of the antibody CB301_H3L1_E6 are shown in SEQ ID NO: 244 to SEQ ID NO: 246, respectively; the amino acid sequences of CDR1 to CDR3 of the heavy chain variable region of the antibody CB301_H3L1_E6 are shown in SEQ ID NO: 247 to SEQ ID NO: 249, respectively; and the amino acid sequences of CDR1 to CDR3 of the light chain variable region of the antibody CB301_H3L1_E6 are shown in SEQ ID NO: 250 to SEQ ID NO: 252, respectively.

The polynucleotide sequences encoding CDR1 to CDR3 of the heavy chain variable region of the antibody CB301_H3L1_F4 are shown in SEQ ID NO: 253 to SEQ ID NO: 255, respectively; the polynucleotide sequences encoding CDR1 to CDR3 of the light chain variable region of the antibody CB301_H3L1_F4 are shown in SEQ ID NO: 256 to SEQ ID NO: 258, respectively; the amino acid sequences of CDR1 to CDR3 of the heavy chain variable region of the antibody CB301_H3L1_F4 are shown in SEQ ID NO: 259 to SEQ ID NO: 261, respectively; and the amino acid sequences of CDR1 to CDR3 of the light chain variable region of the antibody CB301_H3L1_F4 are shown in SEQ ID NO: 262 to SEQ ID NO: 264, respectively.

The polynucleotide sequences encoding CDR1 to CDR3 of the heavy chain variable region of the antibody CB301_H3L1_G11 are shown in SEQ ID NO: 265 to SEQ ID NO: 267, respectively; the polynucleotide sequences encoding CDR1 to CDR3 of the light chain variable region of the antibody CB301_H3L1_G11 are shown in SEQ ID NO: 268 to SEQ ID NO: 270, respectively; the amino acid sequences of CDR1 to CDR3 of the heavy chain variable region of the antibody CB301_H3L1_G11 are shown in SEQ ID NO: 271 to SEQ ID NO: 273, respectively; and the amino acid sequences of CDR1 to CDR3 of the light chain variable region of the antibody CB301_H3L1_G11 are shown in SEQ ID NO: 274 to SEQ ID NO: 276, respectively.

The polynucleotide sequences encoding CDR1 to CDR3 of the heavy chain variable region of the antibody CB301_OPALTL_B5 are shown in SEQ ID NO: 277 to SEQ ID NO: 279, respectively; the polynucleotide sequences encoding CDR1 to CDR3 of the light chain variable region of the antibody CB301_OPALTL_B5 are shown in SEQ ID NO: 280 to SEQ ID NO: 282, respectively; the amino acid sequences of CDR1 to CDR3 of the heavy chain variable region of the antibody CB301_OPALTL_B5 are shown in SEQ ID NO: 283 to SEQ ID NO: 285, respectively; and the amino acid sequences of CDR1 to CDR3 of the light chain variable region of the antibody CB301_OPALTL_B5 are shown in SEQ ID NO: 286 to SEQ ID NO: 288, respectively.

The polynucleotide sequences encoding CDR1 to CDR3 of the heavy chain variable region of the antibody CB301_OPALTL_E6 are shown in SEQ ID NO: 289 to SEQ ID NO: 291, respectively; the polynucleotide sequences encoding CDR1 to CDR3 of the light chain variable region of the antibody CB301_OPALTL_E6 are shown in SEQ ID NO: 292 to SEQ ID NO: 294, respectively; the amino acid sequences of CDR1 to CDR3 of the heavy chain variable region of the antibody CB301_OPALTL_E6 are shown in SEQ ID NO: 295 to SEQ ID NO: 297, respectively; and the amino acid sequences of CDR1 to CDR3 of the light chain variable region of the antibody CB301_OPALTL_E6 are SEQ ID NO: 298 to SEQ ID NO: 300, respectively.

Further, the polynucleotide sequence encoding the heavy chain variable region of the antibody CK1, the polynucleotide sequence encoding the light chain variable region of the antibody CK1, the amino acid sequence of the heavy chain variable region of the antibody CK1, and the amino acid sequence of the light chain variable region of the antibody CK1 are shown in SEQ ID NOS: 301 to 304, respectively.

The polynucleotide sequence encoding the heavy chain variable region of the antibody CK2, the polynucleotide sequence encoding the light chain variable region of the antibody CK2, the amino acid sequence of the heavy chain variable region of the antibody CK2, and the amino acid sequence of the light chain variable region of the antibody CK2 are shown in SEQ ID NOS: 305 to 308, respectively.

The polynucleotide sequence encoding the heavy chain variable region of the antibody CK3, the polynucleotide sequence encoding the light chain variable region of the antibody CK3, the amino acid sequence of the heavy chain variable region of the antibody CK3, and the amino acid sequence of the light chain variable region of the antibody CK3 are shown in SEQ ID NOS: 309 to 312, respectively.

The polynucleotide sequence encoding the heavy chain variable region of the antibody CL4, the polynucleotide sequence encoding the light chain variable region of the antibody CL4, the amino acid sequence of the heavy chain variable region of the antibody CL4, and the amino acid sequence of the light chain variable region of the antibody CL4 are shown in SEQ ID NOS: 313 to 316, respectively.

The polynucleotide sequence encoding the heavy chain variable region of the antibody CL5, the polynucleotide sequence encoding the light chain variable region of the antibody CL5, the amino acid sequence of the heavy chain variable region of the antibody CL5, and the amino acid sequence of the light chain variable region of the antibody CL5 are shown in SEQ ID NOS: 317 to 320, respectively.

The polynucleotide sequence encoding the heavy chain variable region of the antibody CL6, the polynucleotide sequence encoding the light chain variable region of the antibody CL6, the amino acid sequence of the heavy chain variable region of the antibody CL6, and the amino acid sequence of the light chain variable region of the antibody CL6 are shown in SEQ ID NOS: 321 to 324, respectively.

The polynucleotide sequence encoding the heavy chain variable region of the antibody CB201, the polynucleotide sequence encoding the light chain variable region of the antibody CB201, the amino acid sequence of the heavy chain variable region of the antibody CB201, and the amino acid sequence of the light chain variable region of the antibody CB201 are shown in SEQ ID NOS: 325 to 328, respectively.

The polynucleotide sequence encoding the heavy chain variable region of the antibody CL8, the polynucleotide sequence encoding the light chain variable region of the antibody CL8, the amino acid sequence of the heavy chain variable region of the antibody CL8, and the amino acid sequence of the light chain variable region of the antibody CL8 are shown in SEQ ID NOS: 329 to 332, respectively.

The polynucleotide sequence encoding the heavy chain variable region of the antibody CL9, the polynucleotide sequence encoding the light chain variable region of the antibody CL9, the amino acid sequence of the heavy chain variable region of the antibody CL9, and the amino acid sequence of the light chain variable region of the antibody CL9 are shown in SEQ ID NOS: 333 to 336, respectively.

The polynucleotide sequence encoding the heavy chain variable region of the antibody CL10, the polynucleotide sequence encoding the light chain variable region of the antibody CL10, the amino acid sequence of the heavy chain variable region of the antibody CL10, and the amino acid sequence of the light chain variable region of the antibody CL10 are shown in SEQ ID NOS: 337 to 340, respectively.

The polynucleotide sequence encoding the heavy chain variable region of the antibody SK11, the polynucleotide sequence encoding the light chain variable region of the antibody SK11, the amino acid sequence of the heavy chain variable region of the antibody SK11, and the amino acid sequence of the light chain variable region of the antibody SK11 are shown in SEQ ID NOS: 341 to 344, respectively.

The polynucleotide sequence encoding the heavy chain variable region of the antibody SK12, the polynucleotide sequence encoding the light chain variable region of the antibody SK12, the amino acid sequence of the heavy chain variable region of the antibody SK12, and the amino acid sequence of the light chain variable region of the antibody SK12 are shown in SEQ ID NOS: 345 to 348, respectively.

The polynucleotide sequence encoding the heavy chain variable region of the antibody SK13, the polynucleotide sequence encoding the light chain variable region of the antibody SK13, the amino acid sequence of the heavy chain variable region of the antibody SK13, and the amino acid sequence of the light chain variable region of the antibody SK13 are shown in SEQ ID NOS: 349 to 352, respectively.

The polynucleotide sequence encoding the heavy chain variable region of the antibody SK14, the polynucleotide sequence encoding the light chain variable region of the antibody SK14, the amino acid sequence of the heavy chain variable region of the antibody SK14, and the amino acid sequence of the light chain variable region of the antibody SK14 are shown in SEQ ID NOS: 353 to 356, respectively.

The polynucleotide sequence encoding the heavy chain variable region of the antibody SK15, the polynucleotide sequence encoding the light chain variable region of the antibody SK15, the amino acid sequence of the heavy chain variable region of the antibody SK15, and the amino acid sequence of the light chain variable region of the antibody SK15 are shown in SEQ ID NOS: 357 to 360, respectively.

The polynucleotide sequence encoding the heavy chain variable region of the antibody SK16, the polynucleotide sequence encoding the light chain variable region of the antibody SK16, the amino acid sequence of the heavy chain variable region of the antibody SK16, and the amino acid sequence of the light chain variable region of the antibody SK16 are shown in SEQ ID NOS: 361 to 364, respectively.

The polynucleotide sequence encoding the heavy chain variable region of the antibody SK17, the polynucleotide sequence encoding the light chain variable region of the antibody SK17, the amino acid sequence of the heavy chain variable region of the antibody SK17, and the amino acid sequence of the light chain variable region of the antibody SK17 are shown in SEQ ID NOS: 365 to 368, respectively.

The polynucleotide sequence encoding the heavy chain variable region of the antibody SL18, the polynucleotide sequence encoding the light chain variable region of the antibody SL18, the amino acid sequence of the heavy chain variable region of the antibody SL18, and the amino acid sequence of the light chain variable region of the antibody SL18 are shown in SEQ ID NOS: 369 to 372, respectively.

The polynucleotide sequence encoding the heavy chain variable region of the antibody CB301_H3L1_A10, the polynucleotide sequence encoding the light chain variable region of the antibody CB301_H3L1_A10, the amino acid sequence of the heavy chain variable region of the antibody CB301_H3L1_A10, and the amino acid sequence of the light chain variable region of the antibody CB301_H3L1_A10 are shown in SEQ ID NOS: 373 to 376, respectively.

The polynucleotide sequence encoding the heavy chain variable region of the antibody CB301_H3L1_A12, the polynucleotide sequence encoding the light chain variable region of the antibody CB301_H3L1_A12, the amino acid sequence of the heavy chain variable region of the antibody CB301_H3L1_A12, and the amino acid sequence of the light chain variable region of the antibody CB301_H3L1_A12 are shown in SEQ ID NOS: 377 to 380, respectively.

The polynucleotide sequence encoding the heavy chain variable region of the antibody CB301_H3L1_E6, the polynucleotide sequence encoding the light chain variable region of the antibody CB301_H3L1_E6, the amino acid sequence of the heavy chain variable region of the antibody CB301_H3L1_E6, and the amino acid sequence of the light chain variable region of the antibody CB301_H3L1_E6 are shown in SEQ ID NOS: 381 to 384, respectively.

The polynucleotide sequence encoding the heavy chain variable region of the antibody CB301_H3L1_F4, the polynucleotide sequence encoding the light chain variable region of the antibody CB301_H3L1_F4, the amino acid sequence of the heavy chain variable region of the antibody CB301_H3L1_F4, and the amino acid sequence of the light chain variable region of the antibody CB301_H3L1_F4 are shown in SEQ ID NOS: 385 to 388, respectively.

The polynucleotide sequence encoding the heavy chain variable region of the antibody CB301_H3L1_G11, the polynucleotide sequence encoding the light chain variable region of the antibody CB301_H3L1_G11, the amino acid sequence of the heavy chain variable region of the antibody CB301_H3L1_G11, and the amino acid sequence of the light chain variable region of the antibody CB301_H3L1_G11 are shown in SEQ ID NOS: 389 to 392, respectively.

The polynucleotide sequence encoding the heavy chain variable region of the antibody CB301_OPALTL_B5, the polynucleotide sequence encoding the light chain variable region of the antibody CB301_OPALTL_B5, the amino acid sequence of the heavy chain variable region of the antibody CB301_OPALTL_B5, and the amino acid sequence of the heavy chain variable region of the antibody CB301_OPALTL_B5 are shown in SEQ ID NOS: 393 to 396, respectively.

The polynucleotide sequence encoding the heavy chain variable region of the antibody CB301_OPALTL_E6, the polynucleotide sequence encoding the light chain variable region of the antibody CB301_OPALTL_E6, the amino acid sequence of the heavy chain variable region of the antibody CB301_OPALTL_E6, and the amino acid sequence of the light chain variable region of the antibody CB301_OPALTL_E6 are shown in SEQ ID NOS: 397 to 400, respectively.

The amino acid sequence of CD300C ECD and the polynucleotide sequence encoding thereof are shown in SEQ ID NOS: 401 and 402, respectively.

One or more of the antibody and an antigen-binding fragment thereof of the present invention may induce the promotion of differentiation into a monocyte-derived macrophage (MDM) or M1 macrophage through engagement with CD300c, but is not limited thereto.

In one embodiment of the previously described embodiments, the anti-CD300c antibody and an antigen-binding fragment thereof can specifically bind to the extracellular domain of the CD300c protein. The extracellular domain of CD300c may be the extracellular domain of the human CD300c protein and include the amino acid sequence represented by SEQ ID NO: 402.

In one embodiment, the antibody and/or an antigen-binding fragment thereof of the present invention have the ability to restore or improve cognitive function in a subject with or at risk of degenerative brain disease. The cognitive functions may include cognitive ability, memory, language ability, visuospatial ability, orientation, executive function, or a combination thereof.

In one embodiment, the antibody of the present invention may have the ability to promote differentiation of monocytes into MDM (Monocyte-derived Macrophage) or M1 macrophages, and the promotion ability may be increased compared to general degenerative brain disease treatments and immunotherapy. As a result, it is expected to show significant preventive or therapeutic effects on degenerative brain disease in patients who have not been cured even by general degenerative brain disease treatments and immunotherapy.

In the present invention, the term "degenerative brain disease" means brain diseases caused by damage to nerve cells and may be accompanied by cognitive dysfunction. The cognitive dysfunction may include cognitive decline, memory impairment, language impairment, visuospatial ability decline, impaired orientation, and executive dysfunction, or a combination thereof.

In one embodiment, the degenerative brain disease may include one or more selected from the group consisting of dementia, Alzheimer's disease, Huntington's disease, Parkinson's disease, cerebral amyloid angiopathy, amyloid stroke, Dutch-type amyloidosis, tauopathies, mild cognitive impairment, corticobasal syndrome, posterior cortical atrophy, primary progressive aphasia, progressive supranuclear palsy, corticobasal degeneration (CBD), amyotrophic lateral sclerosis, Creutzfeldt-Jakob disease, amnesia, learning disabilities, and memory impairment, but is not limited thereto.

The dementia refers to the decline or gradual decline of cognitive function caused by brain damage or disease and may generally include disorientation, memory, judgment, and intelligence impairment, emotional instability, or a combination thereof. The dementia may include one or more selected from the group consisting of Alzheimer's disease dementia, Huntington's disease dementia, Parkinson's disease dementia, vascular dementia, neuroinflammatory dementia, cerebral infarction dementia, senile dementia, dementia with Lewy bodies (DLB), multi-infarct dementia (MID), frontotemporal lobar degeneration (FTLD), Creutzfeldt-Jakob disease dementia, Pick's disease dementia, corticobasal ganglia degeneration dementia, normal pressure hydrocephalus dementia, and dementia caused by head trauma, but is not limited thereto.

In one embodiment, the degenerative brain disease may be Alzheimer's disease.

In one embodiment of the present invention, it was confirmed through the Y-maze test using 5xFAD mice induced with degenerative brain disease that the reduced alteration behavior due to the induction of degenerative brain disease was statistically significantly recovered by the administration of the anti-CD300c antibody.

Further, in another embodiment of the present invention, it was confirmed through the Morris water maze test that the reduced cognitive ability and memory of the 5xFAD mice induced with degenerative brain disease due to the induction of degenerative brain disease were statistically significantly recovered by the administration of the anti-CD300c antibody.

Furthermore, in another embodiment of the present invention, it was confirmed that an anti-CD300c antibody of the present invention has the ability to promote differentiation of monocytes to a monocyte-derived macrophage (MDM) or M1 macrophage. The degenerative brain disease of the present invention may be prevented or treated by promoting differentiation of monocytes into MDM or M1 macrophage, and such results suggest that an anti-CD300c antibody and/or an antigen-binding fragment thereof of the present invention may also treat various degenerative brain disease (*e.g.,* Huntington's disease and Parkinson's disease) by promoting differentiation into MDM or M1 macrophage.

The composition of the present invention is for preventing or treating degenerative brain disease, and any one or more of the antibody and an antigen-binding fragment thereof included therein may be for recovering or improving the cognitive function of a subject having or at risk of degenerative brain disease.

In the present invention, the term "treatment" generally means obtaining the desired pharmacological and/or physiological effect and may include all actions in which the symptoms of degenerative brain disease are improved, delayed, or beneficially altered by the administration of the composition of the present invention. Further, due to the nature of brain diseases, actions that maintain the current state without worsening the symptoms may also be included in the treatment. Such effects have therapeutic effects in terms of partially or completely curing the disease and/or side effects caused by such diseases. The desired therapeutic effects include prevention of occurrence or recurrence of the disease, improvement of symptoms, reduction of any direct or indirect pathological consequences of the disease, reduction of the progression rate of the disease, improvement or alleviation of the disease conditions, and remission or improved prognosis, but are not limited to.

In the present invention, the term "prevention" refers to obtaining preventive treatment, *i.e.,* obtaining the effect for preventing the disease rather than treating the same. The prevention means obtaining the desired preventive pharmacological and/or physiological effect in terms of partially or completely preventing the disease or symptoms thereof.

The pharmaceutical composition may further comprise a pharmaceutically acceptable carrier.

In the present invention, the term "pharmaceutically acceptable carrier" refers to a carrier or diluent that does not stimulate the organism and does not impair the biological activity and properties of the administered compound. Pharmaceutically acceptable carriers for compositions formulated as liquid solutions include those that are sterile and biocompatible, such as saline, sterile water, Ringer's solution, buffered saline, albumin injection solution, dextrose solution, maltodextrin solution, glycerol, ethanol, and mixtures of one or more of these components, and other conventional additives, such as antioxidants, buffers, and bacteriostats, may be added as needed. Further, diluents, dispersants, surfactants, binders, and lubricants may be further added to formulate the composition into injectable formulations (*e.g.,* aqueous solutions, suspensions, emulsions, *etc*.), pills, capsules, granules, or tablets.

The pharmaceutical composition may be in various formulations for oral or parenteral administration. When formulated, it is prepared using commonly used fillers, extenders, binders, wetting agents, disintegrants, surfactants, diluents or excipients. Solid formulations for oral administration include tablets, pills, powders, granules, capsules, *etc.,* which are prepared by mixing one or more compounds with at least one or more excipients, *e.g.,* starch, calcium carbonate, sucrose or lactose, gelatin, *etc.* Further, in addition to simple excipients, lubricants such as magnesium stearate, talc, and the like may also be used. Liquid formulations for oral administration include suspensions, oral liquids, emulsions, syrups, *etc.,* which may contain various excipients, *e.g.,* wetting agents, sweeteners, flavoring agents, preservatives, *etc.,* in addition to simple diluents such as water and liquid paraffin. Formulations for parenteral administration include, for example, sterile aqueous solutions, non-aqueous solvents, suspensions, emulsions, lyophilized preparations, and suppositories. Non-aqueous solutions and suspensions may include propylene glycol, polyethylene glycol, vegetable oils such as olive oil, injectable esters such as ethyl oleate, *etc.* Bases for suppositories may include witepsol, macrogol, tween 61, cacao butter, laurin butter, glycerogelatin, *etc.*

The pharmaceutical composition may have any one formulation selected from the group consisting of tablets, pills, powders, granules, capsules, suspensions, oral liquids, emulsions, syrups, sterile aqueous solutions, non-aqueous solvents, lyophilized preparations, and suppositories.

The pharmaceutical composition of the present invention may be administered in a pharmaceutically effective amount to a subject.

In the present invention, the term "pharmaceutically effective amount" means an amount sufficient to treat a disease at a reasonable benefit-risk ratio applicable to medical treatment, and the effective dose level may be determined based on factors including the type, severity, age, and sex of subject, type of brain disease, activity of the drug, sensitivity to the drug, administration time, administration route and excretion rate, treatment period, and concomitant medications, and other factors well known in the medical field. The composition of the present invention may be administered as a single agent or in combination with other therapeutic agents, and be administered sequentially or simultaneously with conventional therapeutic agents. It may also be administered in single or multiple doses. It is important to administer an amount that can maximize the effect with minimal side effects considering all these factors, and this can easily be determined by those skilled in the art.

In the present invention, the term "subject" is used interchangeably with "patient" and "individual," and may refer to a mammal in need of prevention or treatment of degenerative brain disease, such as primates (*e.g.,* humans), pets (*e.g.,* dogs, cats, *etc*.), livestock (*e.g.,* cattle, pigs, horses, sheep, goats, *etc*.), and laboratory animals (*e.g.,* rats, mice, guinea pigs, *etc.).* In one embodiment of the present invention, the subject may be a non-human or a human.

Another aspect of the present invention provides a method for preventing or treating degenerative brain disease, comprising administering one or more of the anti-CD300c antibody and an antigen-binding fragment thereof to a subject having or at risk of degenerative brain disease.

The anti-CD300c antibody, antigen-binding fragment thereof, degenerative brain disease, subject, prevention, and treatment are as described in other aspects above.

In the present invention, the term "administration" means providing a substance (*e.g.,* one or more of the anti-CD300c antibody and an antigen-binding fragment thereof) to a subject to achieve a preventive or therapeutic purpose (*e.g.,* prevention or treatment of degenerative brain disease).

The antibody and/or antigen-binding fragment of the present invention may be administered in various ways depending on whether local or systemic treatment is desired and the area to be treated. The method of administering the antibody and/or an antigen-binding fragment of the present invention to a subject can easily be determined by those skilled in the art. The route of administration may be oral, parenteral, inhalation, or topical. For example, parenteral administration may include intravenous administration, subcutaneous administration, intraperitoneal administration, intramuscular administration, rectal administration, vaginal administration, intrathecal administration, intraventricular administration, or intracerebroventricular administration, but is not limited thereto. In one embodiment, the antibody and/or an antigen-binding fragment of the present invention may be delivered across the blood-brain barrier (BBB) using various suitable compositions and methods described herein or known in the art.

The effective amount or effective non-toxic amount of the antibody and/or an antigen-binding fragment of the present invention may be determined by conventional experiments. For example, the therapeutic active amount of the antibody and/or an antigen-binding fragment of the present invention may vary depending on factors such as the stage of the disease, the severity of the disease, the age, sex, medical complications, and weight of the subject, and the ability of the antibody to elicit the desired response in the subject. The dose and route of administration of the antibody and/or an antigen-binding fragment of the present invention may be adjusted to provide an optimal therapeutic response. For example, several divided doses may be administered daily, weekly, biweekly, every three weeks, every four weeks, *etc.*, and/or the dose may be proportionally reduced or increased depending on the urgency of the treatment situation.

The antibody and/or an antigen-binding fragment of the present invention may be administered in combination with one or more other agents effective for preventing or treating degenerative brain disease. Such other agents include any agents that may improve or ameliorate degenerative brain disease, such as compounds, gene therapies, proteins (including antibodies), but are not limited thereto. When the antibody and/or antigen-binding fragment of the present invention is administered in combination with other agents, they may be formulated as a single composition for simultaneous delivery or as two or more compositions (*e.g*., kits) for separate formulation. Each component may be administered to the subject at different times from when the other component is administered. In a specific embodiment, each administration may be given at multiple intervals over a given period non-simultaneously (*e.g*., individually or sequentially). Further, individual ingredients may be administered to the subject by the same or different routes. The pharmaceutical composition of the present invention may be used in combination with one or more known treatments for degenerative brain disease.

Another aspect of the present invention provides a food composition for preventing or improving degenerative brain disease, comprising one or more of an anti-CD300c antibody and an antigen-binding fragment thereof.

The anti-CD300c antibody, antigen-binding fragment thereof, degenerative brain disease, and prevention are as described in other aspects above.

The food composition may be a health functional food.

In the present invention, the term "improvement" means all actions in which the survival, recurrence, and/or therapeutic resistance of a subject suspected of having or having a degenerative brain disease is reduced, or the symptoms are improved or beneficially altered, using one or more of the anti-CD300c antibody and an antigen-binding fragment thereof.

In the present invention, the term "food" includes all conventional meanings of food, such as meats, sausages, bread, chocolate, candies, snacks, confectionery, pizza, ramen, other noodles, gums, dairy products including ice cream, various soups, beverages, teas, drinks, alcoholic beverages, vitamin complexes, and health functional foods, and is not limited thereto as long as the food can include one or more of the anti-CD300c antibody and an antigen-binding fragment thereof of the present invention. When manufacturing the food composition, raw materials and ingredients commonly added in the art may be added to manufacture the food composition, and the type is not particularly limited. For example, as in conventional foods, various herbal extracts, sitologically acceptable food additives, natural carbohydrates, *etc.* may be included as additional ingredients, but are not limited thereto. The mixing amounts of the active ingredients may be appropriately determined according to the intended use.

In the present invention, the term "health functional food" is synonymous with food for special health use (FoSHU) and refers to foods manufactured and processed for health supplementation purposes, using specific ingredients as the raw materials or by using methods such as extracting, concentrating, purifying, mixing, *etc.* of specific ingredients contained in food materials. These foods refer to foods designed and processed to sufficiently exert biological regulatory functions such as biological defense, regulation of biological rhythms, and prevention and recovery from diseases in the body by the ingredients. The composition for health functional foods may perform functions related to disease prevention, disease recovery, *etc.* The health functional food of the present invention may be used interchangeably with terms known in the art, such as functional foods.

Another aspect of the present invention provides a feed composition for preventing or improving degenerative brain disease, comprising one or more of an anti-CD300c antibody and an antigen-binding fragment thereof.

The anti-CD300c antibody, antigen-binding fragment thereof, degenerative brain disease, prevention, and improvement are as described in other aspects above.

The feed composition may be for animals other than humans, and may be for the subject above, such as mammals and birds including cattle, pigs, sheep, chickens, dogs, and humans, but is not limited thereto.

The feed composition may comprise known carriers, stabilizers, or additives acceptable for pharmaceutical, sitological, or feed use, in addition to the anti-CD300c antibody and an antigen-binding fragment thereof of the present invention. Examples include binders, emulsifiers, preservatives, *etc.*, which are added to prevent quality deterioration, and amino acids, vitamins, enzymes, flavoring agents, non-protein nitrogen compounds, silicates, buffers, extracts, oligosaccharides, *etc.,* which are added to a feed to enhance efficacy. Additionally, feed mixtures, *etc.* may be included, but are not limited thereto. The feed composition may also include various nutrients, such as vitamins, amino acids, and minerals, antioxidants, and other additives, as needed, and may be in a suitable form such as powder, granules, pellets, suspensions, *etc.* The feed composition of the present invention may be supplied alone or mixed with feed for individual animals. The feed of the present invention is not particularly limited and may be any feed such as powdered feed, solid feed, dry feed, wet feed, moist pellet feed, dry pellet feed, extruder pellet (EP) feed, raw feed, *etc.*

Another aspect of the present invention provides a use of the anti-CD300c antibody and/or an antigen-binding fragment thereof for preventing or treating degenerative brain disease.

In one embodiment, the degenerative brain disease may be prevented or treated by promoting differentiation of monocytes into MDM or M1 macrophage.

Another aspect of the present invention provides a use of an anti-CD300c antibody and/or an antigen-binding fragment thereof for manufacturing a composition (e.g., a drug) for preventing or treating degenerative brain disease.

The anti-CD300c antibody, antigen-binding fragment thereof, degenerative brain disease, prevention, and treatment are as described in other aspects above.

Another aspect of the present invention provides a method for promoting differentiation of monocytes into MDM or M1 macrophage, comprising administering one or more of an anti-CD300c antibody and an antigen-binding fragment thereof to a subject; or treating the same to isolated cells.

The subject may be a subject having or at risk of degenerative brain disease, but is not limited thereto.

Another aspect of the present invention provides the use of an anti-CD300c antibody and/or an antigen-binding fragment thereof in promoting differentiation into MDM or M1 macrophage.

The method and/or use for promoting differentiation into MDM or M1 macrophage may be *in vitro* or *in vivo.*

The anti-CD300c antibody, antigen-binding fragment thereof, subject, *etc.* are as described in other aspects above.

### [Mode for Carrying Out the Invention]

Hereinafter, the present invention will be described in detail by way of specific Examples. However, these Examples are merely preferred embodiments for illustrating the present invention, and thus, the scope of the present invention is not intended to be limited thereto. Meanwhile, technical matters not described in this specification can be sufficiently understood and readily implemented by those skilled in the art or in related technical fields.

### Example 1. Preparation of anti-CD300c antibodies (CB201, CL10, and SL18)

An antibody expression vector with separated heavy and light chains was constructed using a base sequence encoding an anti-CD300c monoclonal antibody that binds specifically to the CD300c antigen with high binding affinity.

To this end, genes were inserted into a pCIW3.3 vector to express the heavy chain and the light chain, respectively, using CB201 CDR sequences (amino acid sequences of CB201 and polynucleotide sequences encoding the same) shown in Table 3, Table 4, and the sequence listing. The vector construction was performed using known methods. The constructed CB201 heavy chain and light chain expression vectors were mixed with polyethylenimine (PEI) at a 1:1 mass ratio and transfected into 293T (human colon cancer cell line) cells to induce antibody expression. On Day 8, the culture was centrifuged to remove the cells and obtain the culture medium. The obtained culture medium was filtered and then resuspended using a mixed solution containing 0.1 M NaH₂PO₄ and 0.1 M Na₂HPO₄ (pH 7.0). The resuspended solution was purified by affinity chromatography using protein A beads (GE Healthcare) and finally eluted using elution buffer (Thermo Fisher).

**[Table 3]**

| Antibo dy | Phage Type | Domain | | DNA Sequence (CDR1, CDR2, and CDR3 are bolded and underlined in the listed order.) | SEQ ID NO: |
|---|---|---|---|---|---|
| CB201 | VH3VL1 | Heavy chain variable region | Full sequence | GAGGTGCAGCTGTTGGAGTC TGGTGGAGGCTTGGTACAGC CTGGAGGTTCTCTTCGCCTC TCCTGTGCAGCCTCCGGATT CACTTTCAGCCGCTACGCAA TGAGCTGGGTCAGACAGGCA CCAGGTAAGGGACTGGAGTG GGTCTCTGCAATTAGCGGTA GCGGTGGTAGCACTTACTAC GCAGACAGCGTGAAGGGTC GCTTCACCATCTCACGCGAC AACTCCAAGAACACCCTGTA CCTGCAGATGAACAGCCTTC GCGCAGAGGACACTGCCGTG TATTACTGCGCACGTAGCAG CCAGGGTATCTTCGACATCT GGGGACAAGGTACTCTGGTC ACTGTCTCCTCA | 325 |
| | | | CDR1 | AGCCGCTACGCAATGAGCTG GGTCAGA | 73 |
| | | | CDR2 | GCAATTAGCGGTAGCGGTG GTAGCACTTACTACGCAGAC | 74 |
| | | | CDR3 | TGCGCACGTAGCAGCCAGG GTATCTTCGACATCTGGGGA | 75 |
| | | Light | Full | CAGTCTGTGCTGACTCAGCC | 326 |
| | | chain variable region | sequence | ACCTTCAGCATCTGGTACTCC AGGTCAGCGCGTCACCATCA GCTGCAGTGGTAACAATATC GGTACTAGACGCGTGCATTG GTATCAGCAACTCCCAGACA CCGCTCCTAAGCTCCTGATTT ACAGTAAGAACAACCGTCCT AGTGGTGTGCCTGATCGCTT TTCTGGGTCCAAGTCTGGCA CCTCAGCCTCTCTGGCTATC AGTGGACTTCGCTCCGAGGA CGAGGCTGACTATTACTGCG CAGCATGGGACGACAGCCT GAGCGGTCCTGTGTTCGGCG GTGGGACCAAACTGACCGTC CTA | |
| | | | CDR1 | TGCAGTGGTAACAATATCGG TACTAGACGCGTG | 76 |
| | | | CDR2 | AAGAACAACCGTCCTAGTGG TGTG | 77 |
| | | | CDR3 | TGCGCAGCATGGGACGACAG CCTGAGCGGTCCTGTGTTC | 78 |

**[Table 4]**

| Antibo dy | Phage Type | Domain | | Amino Acid Sequence (CDR1, CDR2, and CDR3 are bolded and underlined in the listed order.) | SEQ ID NO: |
|---|---|---|---|---|---|
| CB201 | VH3VL1 | Heavy | Full | EVQLLESGGGLVQPGGSLRLS | 327 |
| | | chain variable region | sequence | CAASGFTFSRYAMSWVRQAP GKGLEWVSAISGSGGSTYYAD SVKGRFTISRDNSKNTLYLQM NSLRAEDTAVYYCARSSQGIF DIWGQGTLVTVSS | |
| | | | CDR1 | **FTFSRYAMSWVR** | 79 |
| | | | CDR2 | **AISGSGGSTYYAD** | 80 |
| | | | CDR3 | **YCARSSQGIFDIW** | 81 |
| | | Light chain variable region | Full sequence | QSVLTQPPSASGTPGQRVTIS CSGNNIGTRRVHWYQQLPDT APKLLIYSKNNRPSGVPDRFS GSKSGTSASLAISGLRSEDEA DYYCAAWDDSLSGPVFGGGT KLTVL | 328 |
| | | | CDR1 | **CSGNNIGTRRVHW** | 82 |
| | | | CDR2 | **SKNNRPSGVP** | 83 |
| | | | CDR3 | **YCAAWDDSLSGPVF** | 84 |

The anti-CD300c monoclonal antibodies CL10 and SL18 were also produced in the same manner as CB201 using Tables 5 to 8.

**[Table 5]**

| Antib ody | Phage Type | Domain | | DNA Sequence (CDR1, CDR2, and CDR3 are bolded and underlined in the listed order.) | SEQ ID NO: |
|---|---|---|---|---|---|
| CL10 | VH3VL1 | Heavy chain variable region | Full sequence | GAGGTGCAGCTGTTGGAGTCT GGTGGAGGCTTGGTACAGCCT GGAGGTTCTCTTCGCCTCTCC TGTGCAG | 337 |
| | | | | CCTCCGGATTCACTTTCAGCA GCTACGGTATGCATTGGGTCA GACAGGCACCAGGTAAGGGA CTGGAGTGGGTCTCTGCAATT AGCGGTAGCGGTGGTAGCAC TTACTACGCAGACAGCGTGAA GGGTCGCTTCACCATCTCACG CGACAACTCCAAGAACACCCT GTACCTGCAGATGAACAGCCT TCGCGCAGAGGACACTGCCGT GTATTACTGCGCAAGCGGTTA CGGTCTGATGGACGTGTGGG GACAAGGTACTCTGGTCACTG TCTCCTCA | |
| | | | CDR1 | TCAGCAGCTACGGTATGCATT GGGTCAGACA | 109 |
| | | | CDR2 | CTGCAATTAGCGGTAGCGGTG GTAGCACTTACTACGCAGACA G | 110 |
| | | | CDR3 | ACTGCGCAAGCGGTTACGGTC TGATGGACGTGTGGGGACA | 111 |
| | | Light chain variable region | Full sequence | TCTGTGCTGACTCAGCCACCT TCAGCATCTGGTACTCCAGGT CAGCGCGTCACCATCAGCTGC ACTCGTAGCAGCGGTATCATC GCAAGCAACTACGTGCAGTG GTATCAGCAACTCCCAGGCAC CGCTCCTAAGCTCCTGATTTA CCGCAACAACCAGCGCCCTA | 338 |
| | | | | GTGGTGTGCCTGATCGCTTTT CTGGGTCCAAGTCTGGCACCT CAGCCTCTCTGGCTATCAGTG GACTTCGCTCCGAGGACGAG GCTGACTATTACTGCAGCAGC TACGCAGGTAACAACAACCT GGTGTTCGGCGGTGGGACCA AACTGACCGTCCTA | |
| | | | DR1 | GCTGCACTCGTAGCAGCGGTA TCATCGCAAGCAACTACGTGC A | 112 |
| | | | CDR2 | CGCAACAACCAGCGCCCTAGT GGTGTG | 113 |
| | | | CDR3 | ACTGCAGCAGCTACGCAGGTA ACAACAACCTGGTGTTCGG | 114 |

**[Table 6]**

| Antibo dy | Phage Type | Domain | | Amino Acid Sequence (CDR1, CDR2, and CDR3 are bolded and underlined in the listed order.) | SEQ ID NO: |
|---|---|---|---|---|---|
| CL10 | VH3VL1 | Heavy chain variable region | Full sequence | EVQLLESGGGLVQPGGSLRLSC AASGFTFSSYGMHWVRQAPGK GLEWVSAISGSGGSTYYADSVK GRFTISRDNSKNTLYLQMNSLRA EDTAVYYCASGYGLMDVWGQG TLVTVSS | 339 |
| | | | CDR1 | **FTFSSYGMHWVR** | 115 |
| | | | CDR2 | **AISGSGGSTYYAD** | 116 |
| | | | CDR3 | **YCASGYGLMDVW** | 117 |
| | | Light chain variable region | Full sequence | QSVLTQPPSASGTPGQRVTISCT RSSGIIASNYVQWYQQLPGTAPK LLIYRNNQRPSGVPDRFSGSKSG TSASLAISGLRSEDEADYYCSSY AGNNNLVFGGGTKLTVL | 340 |
| | | | CDR1 | **CTRSSGIIASNYVQW** | 118 |
| | | | CDR2 | **RNNQRPSGVP** | 119 |
| | | | CDR3 | **YCSSYAGNNNLVF** | 120 |

**[Table 7]**

| Anti-b ody | Phage Type | Domain | | DNA Sequence(CDR1, CDR2, AND CDR3 are bolded and underlined in the listed order.) | SEQ ID NO: |
|---|---|---|---|---|---|
| SL18 | Lambda | Heavy chain variable region | Full sequence | CGAGTGCAGCTGCTGGAAAGT GGAGGTGGACTGGTGCAGCC TGGCGGCAGCCTGCGCCTGA GCTGTGCCG CCAGCGGATTCACCTTCAGCG ATTATCATATGCATTGGGTTC GCCAAGCACCTGGCAAAGGC CTGGAATGGGTGAGCACCATC AGCAGCAGCGGCGGCTATAC CTATTATGCCGAAAGCGTGAA AAGCCGCTTTACCATCAGCCG CGATAACAGCAAAAACACCCT GTATCTGCAGATGAACAGCCT GCGCGCCGAGGACACCGCAG TCTACTACTGTGCCCGATCGA | 369 |
| | | | | TACGCCTGCCTCTGGATTATT GGGGACAAGGTACTCTGGTGA CCGTGAGCAG CA | |
| | | | CDR1 | TCACCTTCAGCGATTATCATAT GCATTGGGTTCG | 205 |
| | | | CDR2 | TCAGCAGCAGCGGCGGCTATA CCTATTATGCCGA | 206 |
| | | | CDR3 | CCCGATCGATACGCCTGCCTC TGGATTATTGGGG | 207 |
| | | Light chain variable region | Full sequence | CAGAGCGTGCTGACCCAGCCT CCTAGCGCCTCCGGTACACCA GGACAGCGCGTGACTATTAGC TGTAGCGGCAACAACATCGG CAGCAAAGGCGTGCATTGGT ATCAGCAACTGCCTGGAACTG CACCTAAGCTGCTGATCTATG AAGATAGCAAACGCCCTAGC GGCGTGCGTGATCGCTTTAGC GGTAGCAAATCAGGCACCAGC GCCAGCCTGGCCATCAGCGG CCTTCGCTCCGAAGATGAAGC CGATTATTATTGTCAGAGCTAT GATAGCACCAAAGGCGTGGT GTTTGGTGGCGGTACCAAGC GACCGTGCTG | 370 |
| | | | CDR1 | GCGGCAACAACATCGGCAGCA AAGGCGTGCATTGGTA | 208 |
| | | | CDR2 | ATGAAGATAGCAAACGCCCTA GCGGCGTGCGTGA | 209 |
| | | | CDR3 | GCTATGATAGCACCAAAGGCG TGGTGTTTGGTGG | 210 |

**[Table 8]**

| Antibo dy | Phage Type | Domain | | Amino Acid Sequence (CDR1, CDR2, and CDR3 are bolded and underlined in the listed order.) | SEQ ID NO: |
|---|---|---|---|---|---|
| SL18 | Lambda | Heavy chain variable region | Full sequence | RVQLLESGGGLVQPGGSLRLSC AASGFTFSDYHMHWVRQAPGKG LEWVSTISSSGGYTYYAESVKSR FTISRDNSKNTLYLQMNSLRAED TAVYYCARSIRLPLDYWGQGTLV TVSS | 371 |
| | | | CDR1 | **FTFSDYHMHWVR** | 211 |
| | | | CDR2 | **TISSSGGYTYYAE** | 212 |
| | | | CDR3 | **YCARSIRLPLDYW** | 213 |
| | | Light chain variable region | Full sequence | QSVLTQPPSASGTPGQRVTISCS GNNIGSKGVHWYQQLPGTAPKL LIYEDSKRPSGVRDRFSGSKSGT SASLAISGLRSEDEADYYCQSYD STKGVVFGGGTKLTVL | 372 |
| | | | CDR1 | **CSGNNIGSKGVHW** | 214 |
| | | | CDR2 | **EDSKRPSGVR** | 215 |
| | | | CDR3 | **YCQSYDSTKGVVF** | 216 |

### Example 2. Confirmation of Binding Affinity of Anti-CD300c Monoclonal Antibody to CD300c Antigen (I): Binding ELISA

The CD300c antigen (250 µg/mL) was diluted to a concentration of 800 ng/mL in coating buffer (0.1 M sodium carbonate, pH 9.0) and 100 µL of the solution was added to each well of a 96-well microplate, followed by overnight incubation at 4°C. The next day, the microplate was washed with 200 µL of PBST three times. Subsequently, 200 µL of blocking buffer (5% skim milk) was added to each well and blocked for 1 hour at room temperature.

The anti-CD300c monoclonal antibody CB201 was diluted to 200 µg/mL in PBS and the concentration was confirmed using Nanodrop (product name: NanoDrop One/Onec). Then, CB201 was serially diluted four-fold from 10 µg/mL in PBS, and 100 µL of each dilution was added to each well, which was then left to react at room temperature for 1 hour and washed with 200 µL of PBST three times. The secondary antibody (conjugated anti-Fc IgG) was diluted 1:10,000 in blocking buffer, and 100 µL was added to each well, followed by incubation at room temperature for 1 hour. The wells were then washed with 200 µL of PBST three times. Subsequently, TMB and hydrogen peroxide were mixed in a 1:1 ratio, and 100 µL of the mixture was added to each well, which was then left to react at room temperature for 7 to 9 minutes. The reaction was stopped by adding 50 µL of 1 N sulfuric acid, and the absorbance at 450 nm was measured using a microplate reader (product name: Varioskan LUX) to obtain the binding affinity results.

Additionally, the binding affinity results for the anti-CD300c monoclonal antibodies CL10 and SL18 were obtained in the same manner as CB201.

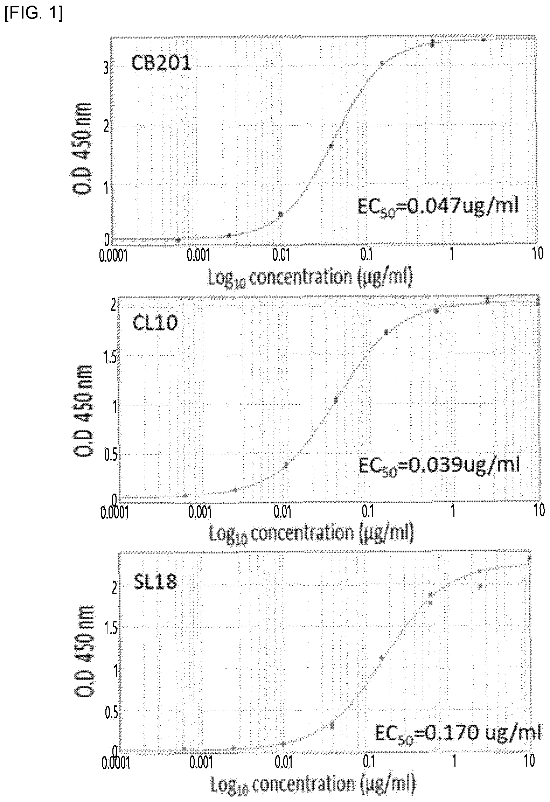

As a result, as shown in FIG. 1, it was confirmed that the anti-CD300c monoclonal antibodies CB201, CL10, and SL18 all bind to CD300c in a concentration-dependent manner, indicating that all of the anti-CD300c monoclonal antibodies CB201, CL10, and SL18 have excellent binding affinity and specificity for the CD300c antigen.

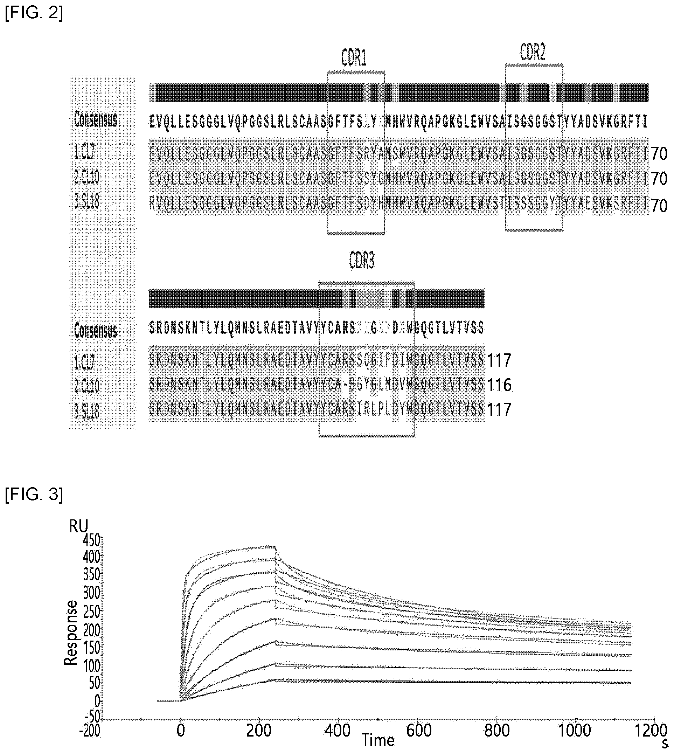

From these results, it was confirmed that the anti-CD300c monoclonal antibodies CB201, CL10, and SL18 not only have excellent binding affinity and specificity for CD300c, but also have similar properties, which is derived from the similarity in the variable region sequences of the three antibodies as shown in FIG. 2.

### Example 3. Confirmation of Binding Affinity of Anti-CD300c Monoclonal Antibody to CD300c Antigen (II): Surface Plasmon Resonance (SPR)

To further confirm the binding affinity between the CD300c antigen and the anti-CD300c monoclonal antibody CB201, a surface plasmon resonance experiment was conducted.

To immobilize CD300c on a CM5 chip, 5 µg/mL of CD300c was diluted in 10 mM acetate buffer (pH 5.5). Then, the flow rate was set to 10 mL/min, and the target resonance unit (RU) was set to 300 RU. The CM5 chip was activated with a mixture of 0.2 M EDC and 0.05 M NHS and blocked with 1 M ethanolamine to immobilize the CM5 chip such that the final RU of CD300c was 399.2 RU. CB201 was then diluted in PBST to concentrations of 0 µg/mL, 0.195 µg/mL, 0.39 µg/mL, 0.78 µg/mL, 1.56 µg/mL, 3.125 µg/mL, and 6.25 µg/mL, and the kinetics/affinity test was performed with a binding time of 240 seconds, a dissociation time of 900 seconds, and a flow rate of 30 µL/min. The surface was then regenerated by flowing 50 mM NaOH over the CM5 chip with immobilized CD300c at a flow rate of 30 µL/min for 30 seconds.

As a result, as shown in FIG. 3, the KD value was analyzed to be 5.199E-10 M, and the binding affinity of the anti-CD300c monoclonal antibody was confirmed to be on a subnanomolar level at 0.52 nM. This indicates high binding affinity of the anti-CD300c monoclonal antibody for the antigen.

### Example 4. Confirmation of Cell Antigen Recognition by Anti-CD300c Monoclonal Antibody

In order to confirm that the anti-CD300c monoclonal antibody (CB201) recognizes cell antigens, FACS binding was performed.

To this end, CD300c was overexpressed in 293T cells (ATCC), and the cells were dispensed into microcentrifuge tubes at 2×10⁵ cells per tube. The anti-CD300c monoclonal antibody was serially diluted three-fold from 10 µg/mL and reacted with the cells in a CO₂ incubator for 30 minutes, followed by washing the tubes twice with FACS buffer. Subsequently, FITC-conjugated anti-human IgG (H+L) diluted 1:100 in FACS buffer was reacted with the anti-CD300c monoclonal antibody bound to the cells in a CO₂ incubator for 30 minutes, followed by washing the tubes twice with FACS buffer. The FITC signal was then measured using a CytoFLEX instrument (Beckman Coulter), and the MFI values were obtained using the CytExpert program. The obtained MFI values were used to plot a sigmoidal curve using the Sigmaplot program to calculate the EC50 value (the effective concentration of a drug that causes 50% of the maximum response). As a result, an EC50 value of 2.7 nM was obtained for 293T cells.

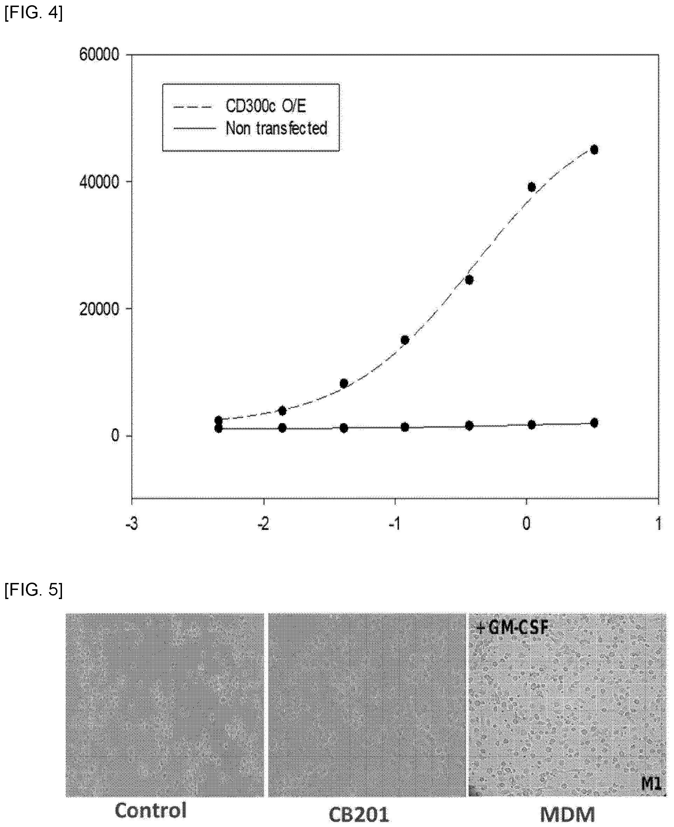

As illustrated in FIG. 4, the anti-CD300c monoclonal antibody was bound to the overexpressed CD300c on the surface of 293T cells with strong affinity, as indicated by the sigmoidal curve obtained from the FACS binding results. Therefore, it was confirmed that the anti-CD300c monoclonal antibody specifically binds to CD300c at the cellular level.

### Example 5. Confirmation of Promotion of Differentiation into Monocyte-derived Macrophage (MDM) (I): Observation of Cell Morphology

To confirm the differentiation pattern of monocytes into monocyte-derived macrophage (MDM) in cell morphology when treated with anti-CD300c monoclonal antibody, THP-1 cells (human monocyte cell line) treated with 10 µg/mL of the anti-CD300c monoclonal antibody CB201 were cultured for 48 hours, and the cell morphology was observed under a microscope.

As a result, as shown in FIG. 5, it was confirmed that the morphology of THP-1 cells in the experimental group (CB201) treated with the anti-CD300c monoclonal antibody changed from suspension cells to round adherent cells, which is the morphology of MDM.

From these results, it was confirmed that the differentiation of monocytes into MDM is promoted by the treatment with the anti-CD300c monoclonal antibody.

### Example 6. Confirmation of Promotion of Differentiation into MDM (II): Cell Signaling

To further confirm that the treatment of THP-1 cells with the anti-CD300c monoclonal antibody CB201 increases the differentiation of monocytes into MDM, the signaling of mitogen-activated protein kinase (MAPK) and NF-kB, which are representative signals of differentiation into MDM, was confirmed.

To this end, THP-1 cells were dispensed into a 6-well plate at 8.8×10⁵ cells/well, and 10 µg/mL of the anti-CD300c monoclonal antibody was added. As a control, the same amount of phosphate-buffered saline (PBS) was added. After culturing for 48 hours, phosphorylated SAPK/JNK, phosphorylated ERK (p44/42), and phosphorylated p38 for MAPK signaling, and phosphorylated NF-kB for NF-kB signaling were confirmed by Western blotting.

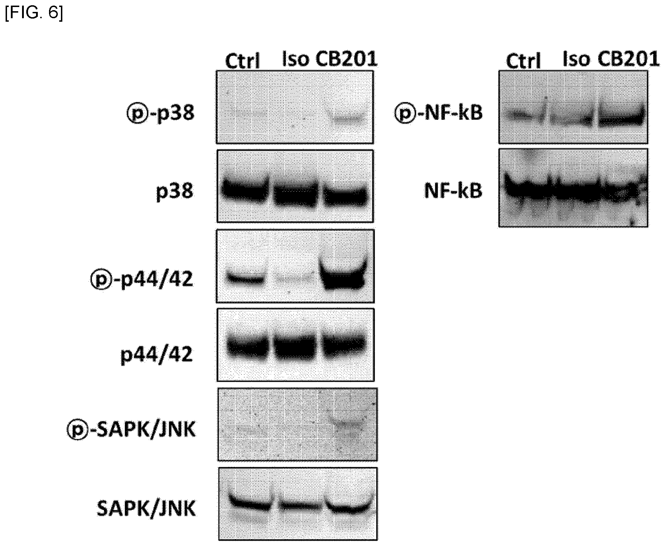

The results of confirming the respective signaling of MAPK and NF-kB are shown in FIG. 6. It was confirmed that the amount of phosphorylated MAPK and NF-kB increased when treated with the anti-CD300c monoclonal antibody compared to the control group.

From these results, it was confirmed that cell signaling for differentiation into MDM increases when treated with the anti-CD300c monoclonal antibody.

### Example 7. Comparison of Differentiation Ability of Anti-CD300c Monoclonal Antibodies into MDM: Measurement of Production of Three Differentiation Markers (TNF-α, IL-1β, and IL-8)

To confirm the differentiation abilities of anti-CD300c monoclonal antibodies and other antibodies into MDM, THP-1 cells were dispensed into a 6-well plate at 8.8×10⁵ cells/well and treated with 10 µg/mL of the anti-CD300c monoclonal antibody CB201. The production of differentiation into MDM markers TNF-α, IL-1β, and IL-8 was confirmed using an ELISA kit. For other antibodies, human IgG Isotype control was treated at a concentration of 10 µg/mL, and the production of TNF-α, IL-1β, and IL-8 was confirmed using an ELISA kit.

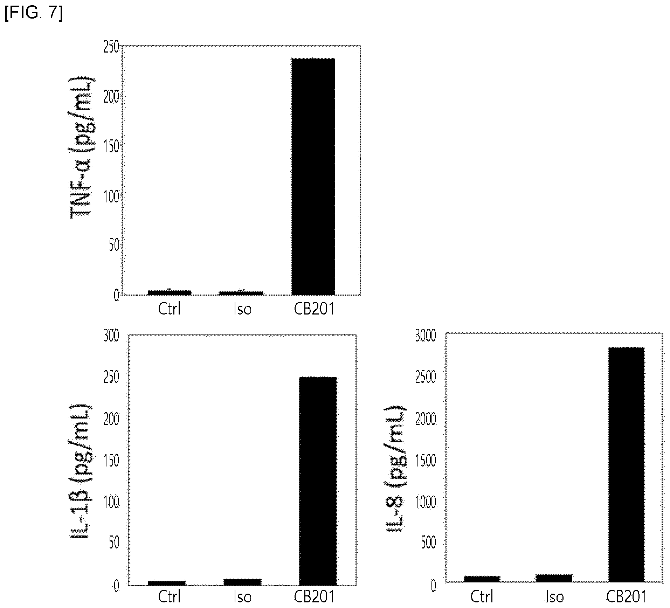

Additionally, the anti-CD300c monoclonal antibodies CL10 and SL18 were also treated to THP-1 cells in the same manner as CB201, and the production of TNF-α was confirmed using an ELISA kit. The results are shown in FIGS. 7 (CB201) and 8 (CL10 and SL18).

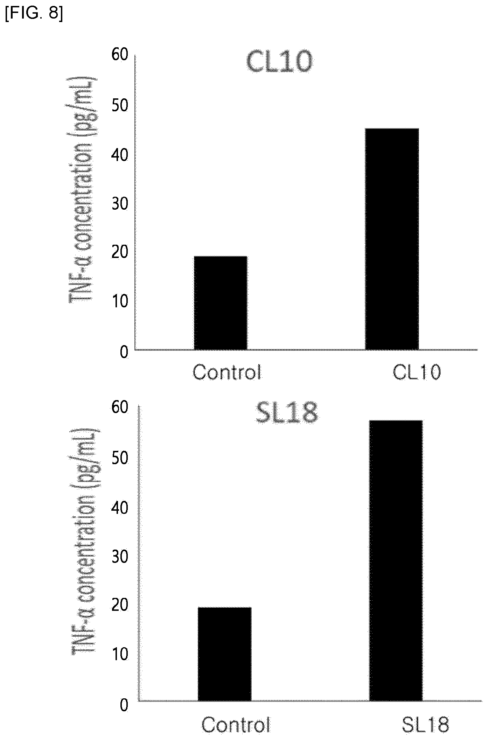

As shown in FIG. 7, it was confirmed that the anti-CD300c monoclonal antibody CB201 significantly increased the production of TNF-α, IL-1β, and IL-8 compared to the control group treated with the Isotype control alone. Similarly, as shown in FIG. 8, it was confirmed that the anti-CD300c monoclonal antibodies CL10 and SL18 significantly increased the production of TNF-α compared to the control group treated with the Isotype control alone. This suggests that anti-CD300c monoclonal antibodies CL10 and SL18 have similar characteristics to CB201.

From these results, it was confirmed that the anti-CD300c monoclonal antibodies significantly increase the differentiation ability of monocytes into MDM.

### Example 8. Confirmation of Promotion of Differentiation Ability of Anti-CD300c Monoclonal Antibody into MDM (III): Confirmation of Extracellular Protein Expression Markers

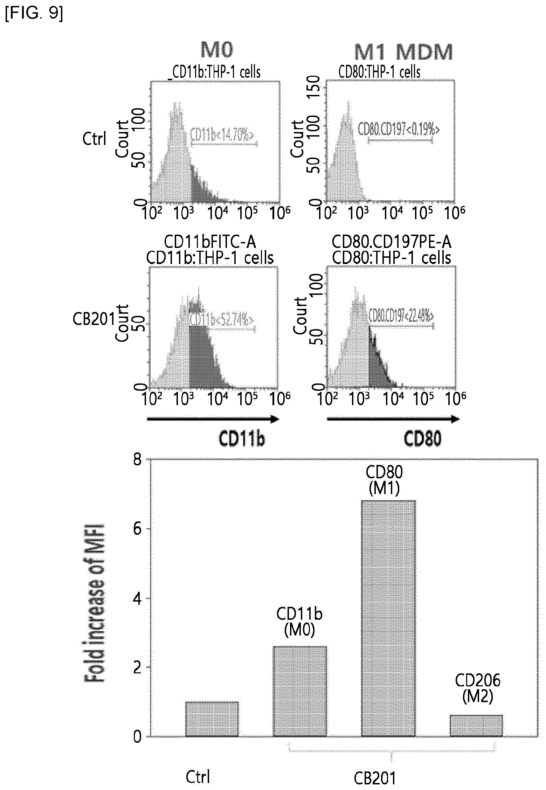

To confirm the differentiation of monocytes into MDMs upon treatment with the anti-CD300c monoclonal antibody CB201, the changes in the expression of differentiation into MDM marker proteins were confirmed through FACS in THP-1 cells treated with CB201 in the same manner as in Example 6. Using antibodies each recognizing the monocyte marker CD11b, the MDM marker CD80, and the M2 macrophage marker CD206, the changes in their expression were confirmed. As a result, as shown in FIG. 9, it was confirmed that the MDM marker CD80 increased in cells treated with CB201.

From these results, it was confirmed that when the anti-CD300c monoclonal antibody CB201 is treated on monocytes, the expression of MDM markers among extracellular proteins increases. This confirmed that CB201 differentiates monocytes into MDM.

### Example 9. Preparation of Degenerative Brain Disease Model Mice

Eight male and eight female 4-8 week-old 5xFAD mice (source: Jackson lab) and three male and three female 6-week-old B6SJLF1/J mice (source: Jackson lab) were purchased, acclimated for about 4-5 months, and observed for general symptoms to confirm their health status. Healthy animals were used for the experiment. The 5xFAD mice, which are modified to express human amyloid precursor protein (APP) and PSEN1 genes causing a total of five AD-related mutations (Swedish (K670N/M671L), Florida (I716V), London (V717I) mutations of APP, and M146L and L286V mutations of PSEN1), were selected as an appropriate model for pathological studies related to neurodegenerative diseases including Alzheimer's symptoms, dementia, *etc.* (Tatsuhiro Ayabe et al., The Neuroscience of Dementia, 2020, Vol. 2, 833-847). The environmental conditions were set at a temperature of 23±3°C, relative humidity of 55±15%, ventilation frequency of 10-20 times/hr, lighting time of 12 hours (lights on at 8 AM and off at 8 PM), and illumination of 150-300 Lux in the third animal breeding area of Notus Co., Ltd. The experimental animals were classified into three groups: a normal mouse group (G1), a mouse group with degenerative brain disease (hereinafter referred to as 5xFAD control group; G2), and a mouse group with degenerative brain disease administered with CB201 (hereinafter referred to as CB201-treated group; G3). The CB201-treated group was administered with CB201 at a dose of 40 mg/kg, and the control groups (normal mouse group and 5xFAD control group) were administered with PBS in the same volume. The administration was performed intraperitoneally twice, on the day of initiation of the administration of the test substance (Day 0) and on the 25th day after the initiation of the administration of the test substance (Day 25). The mouse groups used in the experiment are shown in Table 9 below.

**[Table 9]**

| Group | Sex | No. of Animals | Anim al No. | Lineag e | Administere d substance | Dose (mg/kg) | Dose (mL/kg) |
|---|---|---|---|---|---|---|---|
| Normal mouse group (G1) | M/F | 3/3 | 1-3/ 12-14 | B6SJL F1/J | Vehicle | - | 10 |
| Degenerative brain disease mouse group (5xFAD control group, G2) | M/F | 4/4 | 4-7/ 15-18 | 5xFAD | Vehicle | - | 10 |
| CB201-treate d group (G3) | M/F | 4/4 | 8-11/ 19-22 | 5xFAD | CB201 | 40 | 10 |

### Example 10. Confirmation of the Effect of Anti-CD300c Monoclonal Antibody on Increasing MDM In Vivo

To confirm whether anti-CD300c monoclonal antibody increases MDM in a mouse model, brain tissues of mice (CB201-treated group; G3) administered with CB201 according to the method of Example 9 were extracted, and the proportion of MDM in brain tissues was confirmed using antibodies against iNOS (i.e., an MDM marker) and CD206 (i.e., an M2 macrophage marker).

Additionally, an experimental group of mice administered with anti-PD-1 antibody was prepared by administering 40 mg/kg of PD-1 antibody instead of 40 mg/kg of CB201 to 5xFAD mice in the same manner as the CB201-treated group in Example 9.

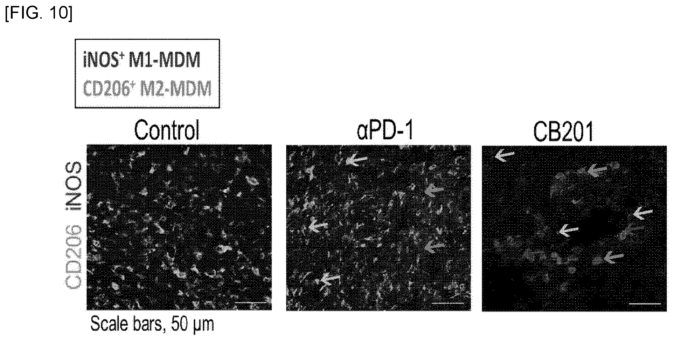

As a result, as shown in FIG. 10, MDM was slightly increased (iNOS was slightly increased) in the brain tissues of the experimental group administered with anti-PD-1 antibody known to have an effect on improving symptoms of degenerative brain disease as an immunotherapy compared to the control group (degenerative brain disease mice in Table 9; G2). However, MDM was significantly increased (iNOS was significantly increased) in the brain tissues of the experimental group administered with anti-CD300c monoclonal antibody (CB201-treated group in Table 9; G3) compared to the control group, and M2 macrophages were hardly observed.

From these results, it was confirmed that the anti-CD300c monoclonal antibody CB201 has a significantly effect on differentiating monocytes into MDM compared to other types of immunotherapy.

### Example 11. Confirmation of Increased Expression of Differentiation into MDM Genes by Anti-CD300c Monoclonal Antibody

In order to confirm the effect of the anti-CD300c monoclonal antibody CB201 on MDM in the brain under *in vivo* conditions, brain tissues of mice in the CB201-treated group (G3 in Table 9) prepared as in Example 9 were extracted, and the cells were stained with a cell viability dye (DAPI), an antibody against the total monocyte marker F4/80, and an antibody against the MDM marker CD11b (source: Invitrogen). The data were then read using a CytoFLEX flow cytometer and analyzed using FlowJo software.

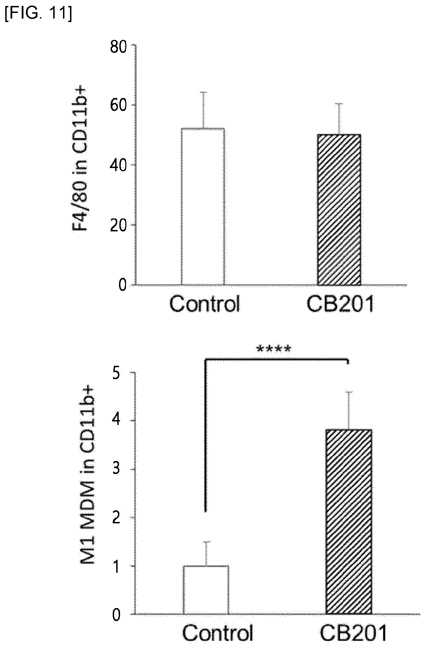

As a result of quantifying each marker relative to viable cells, as shown in FIG. 11, it was confirmed that the expression of the monocyte marker F4/80 in the brain tissues of mice treated with anti-CD300c monoclonal antibody alone was similar compared to the control group, while the expression of the differentiation into MDM marker CD11b was increased (corresponding to M1 MDM in CD11b+ in FIG. 11).

As described below, it was confirmed that the anti-CD300c monoclonal antibody has a therapeutic effect on degenerative brain disease, and these results suggest that the therapeutic effect of the anti-CD300c monoclonal antibody on degenerative brain disease is due to the increased differentiation of MDM in brain tissues through the administration of the anti-CD300c monoclonal antibody.

### Example 12. Nano-string Immune Profiling

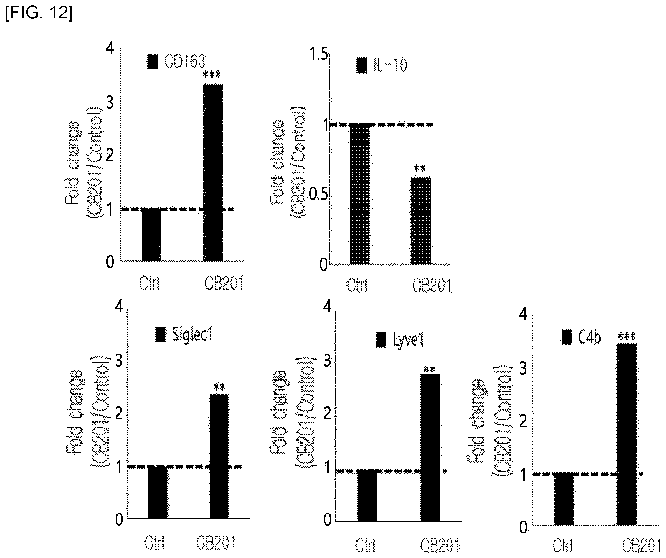

In order to confirm the changes in immune cells and brain tissue environment in a mouse model with degenerative brain disease when administered with the anti-CD300c monoclonal antibody (CB201), brain tissues were extracted from the CB201-treated group (G3 in Table 9) prepared as in Example 9. From these tissues, RNA was extracted and purified, and changes in monocyte markers were confirmed through nano-string immune profiling, which is a known method. The results of observing in comparison with the control group are shown in FIG. 12.

As shown in FIG. 12, it was confirmed that the expression of differentiation into MDM markers CD163, Siglec1, Lyve1, and C4b increased upon administration of the anti-CD300c monoclonal antibody, while the expression of IL-10, which exacerbates the progression of degenerative brain disease in the brain, decreased. This confirmed that MDMs in brain tissues were differentiated, and the immune system of the brain changed in a direction that treats degenerative brain disease.

### Example 13. Y-Maze Test

Six-week-old 5xFAD mice were purchased and acclimated for about five months to create a degenerative brain disease model exhibiting symptoms related to neurodegenerative diseases, including degenerative brain disease symptoms.

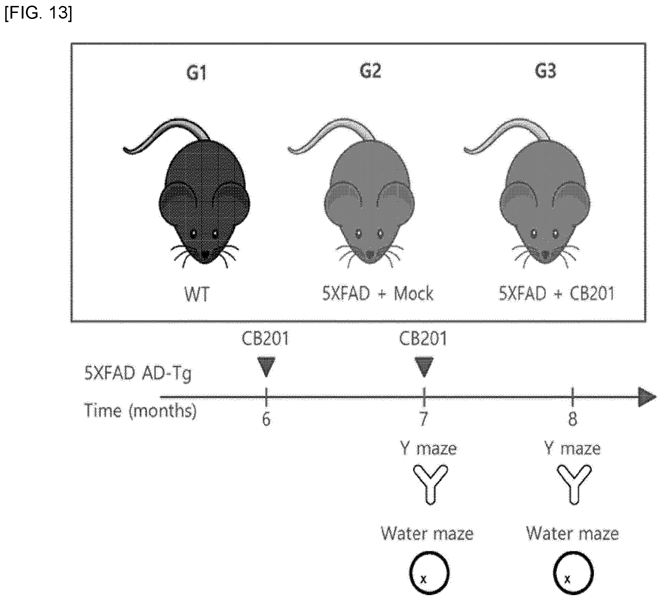

Subsequently, 40 mpk of anti-CD300c monoclonal antibody (CB201) was administered intraperitoneally (I.P) to the degenerative brain disease model, followed by another administration on Day 28, and the Y-maze test was performed on Day 31. The control group was injected with the same amount of PBS. The experimental timeline is shown in FIG. 13.

The experimental apparatus for the Y-maze test consisted of a Y-shaped maze made of black acrylic plates (10 cm × 41 cm × 25 cm), with each maze designated as areas A, B, and C. The experimental animals were placed in a random area and allowed to move freely for 7 minutes. The number of entries and the order of each experimental animal into each maze were measured to evaluate spontaneous alteration (%). When the experimental animal entered three different areas sequentially, it was recognized as 1 point (alteration, in the order of ABC, BCA, CAB, *etc.*), whereas no point was given when the animals entered non-sequentially. Spontaneous alteration (%) was calculated using the following equation: Spontaneous alteration (%) = actual alteration/maximum alteration (total entries - 2) × 100

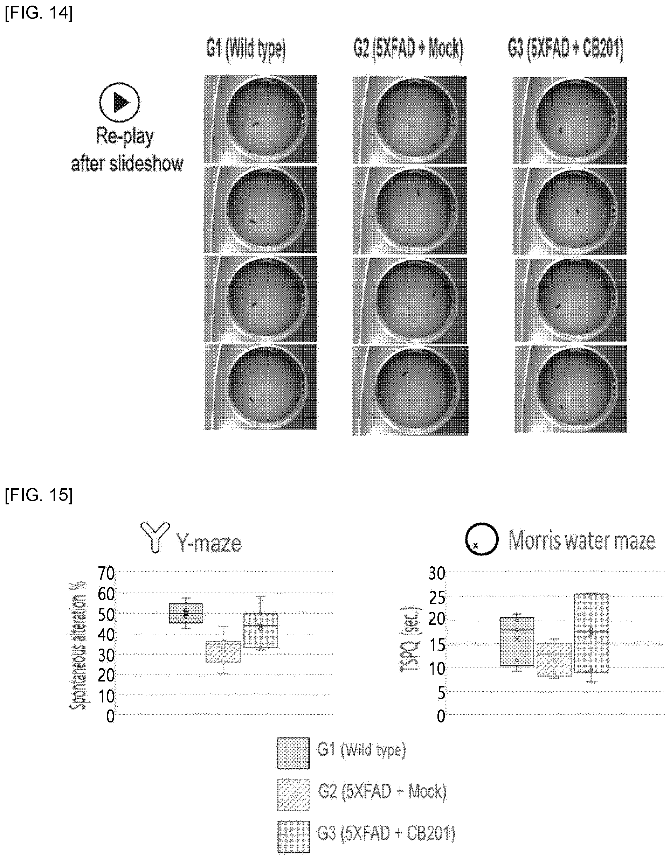

As a result, as shown in FIG. 15, the experimental results confirmed that spontaneous alteration decreased in the degenerative brain disease mouse model (G2; 5XFAD+Mock) compared to normal mice (G1; wild-type), whereas spontaneous alteration recovered to 87% of the level of normal mice (G1; wild-type) when CB201 was administered to the degenerative brain disease mouse model (G3; 5XFAD+CB201). These results suggest that the anti-CD300c monoclonal antibody may serve as a drug that alleviates symptoms such as cognitive decline and decreased spontaneous alteration caused by degenerative brain disease.

### Example 14. Morris Water Maze Test

CB201 was administered to mice of the degenerative brain disease model in the same manner as in Example 13, and a Morris water maze test was performed on Days 35 to 40. The experimental equipment consisted of a circular tank (stainless steel, diameter 150 cm, height 45 cm), an escape platform (diameter 10 cm, height 30 cm), and four markers attached to the walls such that the location of the escape platform may be remembered. The tank was filled with water at a temperature of about 22 ± 2°C, and the escape platform was installed 1 cm above the water surface or 0.5 cm to 1.5 cm below the water surface according to the experimental purpose. When placed below the water surface, the water was made opaque with white water-based paint so that the escape platform was not visible to the naked eye. The water maze was divided into quadrants: northeast (NE), northwest (NW), southeast (SE), and southwest (SW). The escape platform was placed in the center of the northeast quadrant, and one of the remaining quadrants was used as the starting position. The experiment was performed over six days, and the starting quadrant and the position of the escape platform for the animals were specified in Table 10 below.

**[Table 10]**

| | Day 1 | | Day 2 | Day 3 | Day 4 | Day 5 | Day 6 |
|---|---|---|---|---|---|---|---|
| | Position of Escape Platform | Starting Direction | Position of Escape Platform: SW, Starting Position: as below | | | | No Escape Platform |
| Trial 1 | SW | South | West | North | North | East | North |
| Trial 2 | NW | North | South | West | East | South | |
| Trial 3 | NE | South | North | East | West | West | |
| Trial 4 | Center | East | East | West | South | East | |
| Trial 5 | SE | West | South | South | North | North | |

| | |
|---|---|
| It should be noted that on Day 1, both the position of the escape platform and the starting direction are changed, whereas on Day 2 to Day 5, the position of the escape platform remains constant while the starting direction is changed. On the Day 6, there is no escape platform, and the trial is performed once. The starting direction on Day 6 is the farthest from the previous position of the escape platform (SW), requiring a certain distance to be traveled before entering the previously-learned escape platform quadrant. | |

On Day 1, cued learning was performed by making the escape platform visible. Each animal was given five trials at 15-second intervals, with a time limit of 60 seconds. If the animals found the escape platform within 60 seconds, they were allowed to stay on the escape platform for 5 seconds before being immediately transported to the rearing cage. If the animals did not find the escape platform within 60 seconds, they were placed on the escape platform for 20 seconds before being immediately transported to the rearing cage.

From Day 2 to Day 5, hidden platform trials, which hide the escape platform by placing it below the water surface, were performed to train the animals to remember the location of the escape platform. Each animal was given five trials at 15-second intervals. The time limit was set to 60 seconds, and if the animals found the escape platform within 60 seconds, they were allowed to stay on the escape platform for 5 seconds before being immediately transported to the rearing cage. If the animal did not find the escape platform within 60 seconds, they were placed on the escape platform for 20 seconds before being immediately transported to the rearing cage.

On Day 6, a prospective randomized open blinded end-point (probe) trial was performed. Without placing the escape platform, the animals were placed in the quadrant farthest from where the platform was placed during the trials from Day 2 to Day 5, and a single trial was performed with a time limit of 60 seconds. The escape latency, which is the time taken for the laboratory animals to find the platform, and the path length taken to find the platform were measured and recorded during the cued learning and hidden platform trials. The swimming speed was calculated by dividing the path length by the escape latency. During the probe trial on Day 6, the time the animal spent in the quadrant where the platform was previously located was recorded.

The previously described Morris water maze test was recorded on video, as shown in FIG. 14. As shown in FIGS. 14 and 15, the experimental results confirmed that the time spent in platform quadrants (TSPQ) decreased in the degenerative brain disease mouse model (G2; 5XFAD+Mock) compared to normal mice (G1; wild-type) while in the group administered with CB201 (G3; 5XFAD+CB201), the TSPQ was recovered to 107% compared to normal mice (G1; wild type). These results confirmed that the administration of CB201 to the disease model mice can restore cognitive and memory abilities in the degenerative brain disease model mice.

### Example 15. Statistical Analysis

Assuming the normality of the data of the test results, the test results were examined using one-way ANOVA. If the results were significant, post-hoc tests were performed using Dunnett's multiple comparison test to analyze significant differences between the test groups. Statistical analysis was performed using Prism 7.04 (GraphPad Software Inc., San Diego, CA, USA), and a p-value of less than 0.05 was considered statistically significant.

From the above description, those of ordinary skill in the art will be able to recognize that the present invention may be implemented in other specific embodiments without modifying its technical idea or essential characteristics. In relation to the above, the above-described embodiments are to be considered only as illustrative in all respects and not restrictive. The scope of the present invention should be construed as including all changes or modifications derived from the meaning and scope of the claims described below and their equivalent concepts rather than the detailed descriptions above.

## Claims

1. A pharmaceutical composition for preventing or treating a degenerative brain disease, comprising one or more of an anti-CD300c antibody and an antigen-binding fragment thereof as active ingredients.

2. The pharmaceutical composition of claim 1, wherein the degenerative brain disease is a disease involving cognitive dysfunction.

3. The pharmaceutical composition of claim 2, wherein the cognitive dysfunction is one or more selected from the group consisting of cognitive decline, memory impairment, language impairment, visuospatial ability decline, impaired orientation, and executive dysfunction.

4. The pharmaceutical composition of claim 1, wherein the degenerative brain disease is one or more selected from the group consisting of dementia, Alzheimer's disease, Huntington's disease, Parkinson's disease, cerebral amyloid angiopathy, amyloid stroke, Dutch-type amyloidosis, tauopathies, mild cognitive impairment, corticobasal syndrome, posterior cortical atrophy, primary progressive aphasia, progressive supranuclear palsy, corticobasal degeneration (CBD), amyotrophic lateral sclerosis, Creutzfeldt-Jakob disease, amnesia, learning disabilities, and memory impairment.

5. The pharmaceutical composition of claim 4, wherein dementia is one or more selected from a group consisting of Alzheimer's disease dementia, Huntington's disease dementia, Parkinson's disease dementia, cerebrovascular dementia, neuroinflammatory dementia, cerebral infarction dementia, senile dementia, dementia with Lewy bodies (DLB), multi-infarct dementia (MID), frontotemporal lobar degeneration (FTLD), Creutzfeldt-Jakob disease dementia, Pick's disease, corticobasal degeneration dementia, dementia due to normal pressure hydrocephalus, and dementia due to head trauma.

6. The pharmaceutical composition of claim 1, wherein the degenerative brain disease comprises Alzheimer's disease.

7. The pharmaceutical composition of claim 1, wherein any one or more of the anti-CD300c antibody and an antigen-binding fragment thereof comprises:
(i) a heavy chain variable region, comprising:
CDR1 consisting of an amino acid sequence selected from the group consisting of SEQ ID NO: 7, SEQ ID NO: 19, SEQ ID NO: 31, SEQ ID NO: 43, SEQ ID NO: 55, SEQ ID NO: 67, SEQ ID NO: 79, SEQ ID NO: 91, SEQ ID NO: 103, SEQ ID NO: 115, SEQ ID NO: 127, SEQ ID NO: 139, SEQ ID NO: 151, SEQ ID NO: 163, SEQ ID NO: 175, SEQ ID NO: 187, SEQ ID NO: 199, SEQ ID NO: 211, SEQ ID NO: 223, SEQ ID NO: 235, SEQ ID NO: 247, SEQ ID NO: 259, SEQ ID NO: 271, SEQ ID NO: 283, and SEQ ID NO: 295;
CDR2 consisting of an amino acid sequence selected from the group consisting of SEQ ID NO: 8, SEQ ID NO: 20, SEQ ID NO: 32, SEQ ID NO: 44, SEQ ID NO: 56, SEQ ID NO: 68, SEQ ID NO: 80, SEQ ID NO: 92, SEQ ID NO: 104, SEQ ID NO: 116, SEQ ID NO: 128, SEQ ID NO: 140, SEQ ID NO: 152, SEQ ID NO: 164, SEQ ID NO: 176, SEQ ID NO: 188, SEQ ID NO: 200, SEQ ID NO: 212, SEQ ID NO: 224, SEQ ID NO: 236, SEQ ID NO: 248, SEQ ID NO: 260, SEQ ID NO: 272, SEQ ID NO: 284, and SEQ ID NO: 296; and
CDR3 consisting of an amino acid sequence selected from the group consisting of SEQ ID NO: 9, SEQ ID NO: 21, SEQ ID NO: 33, SEQ ID NO: 45, SEQ ID NO: 57, SEQ ID NO: 69, SEQ ID NO: 81, SEQ ID NO: 93, SEQ ID NO: 105, SEQ ID NO: 117, SEQ ID NO: 129, SEQ ID NO: 141, SEQ ID NO: 153, SEQ ID NO: 165, SEQ ID NO: 177, SEQ ID NO: 189, SEQ ID NO: 201, SEQ ID NO: 213, SEQ ID NO: 225, SEQ ID NO: 237, SEQ ID NO: 249, SEQ ID NO: 261, SEQ ID NO: 273, SEQ ID NO: 285, and SEQ ID NO: 297; and
(ii) a light chain variable region, comprising:
CDR1 consisting of an amino acid sequence selected from the group consisting of SEQ ID NO: 10, SEQ ID NO: 22, SEQ ID NO: 34, SEQ ID NO: 46, SEQ ID NO: 58, SEQ ID NO: 70, SEQ ID NO: 82, SEQ ID NO: 94, SEQ ID NO: 106, SEQ ID NO: 118, SEQ ID NO: 130, SEQ ID NO: 142, SEQ ID NO: 154, SEQ ID NO: 166, SEQ ID NO: 178, SEQ ID NO: 190, SEQ ID NO: 202, SEQ ID NO: 214, SEQ ID NO: 226, SEQ ID NO: 238, SEQ ID NO: 250, SEQ ID NO: 262, SEQ ID NO: 274, SEQ ID NO: 286, and SEQ ID NO: 298;
CDR2 consisting of an amino acid sequence selected from the group consisting of SEQ ID NO: 11, SEQ ID NO: 23, SEQ ID NO: 35, SEQ ID NO: 47, SEQ ID NO: 59, SEQ ID NO: 71, SEQ ID NO: 83, SEQ ID NO: 95, SEQ ID NO: 107, SEQ ID NO: 119, SEQ ID NO: 131, SEQ ID NO: 143, SEQ ID NO: 155, SEQ ID NO: 167, SEQ ID NO: 179, SEQ ID NO: 191, SEQ ID NO: 203, SEQ ID NO: 215, SEQ ID NO: 227, SEQ ID NO: 239, SEQ ID NO: 251, SEQ ID NO: 263, SEQ ID NO: 275, SEQ ID NO: 287, and SEQ ID NO: 299; and
CDR3 consisting of an amino acid sequence selected from the group consisting of SEQ ID NO: 12, SEQ ID NO: 24, SEQ ID NO: 36, SEQ ID NO: 48, SEQ ID NO: 60, SEQ ID NO: 72, SEQ ID NO: 84, SEQ ID NO: 96, SEQ ID NO: 108, SEQ ID NO: 120, SEQ ID NO: 132, SEQ ID NO: 144, SEQ ID NO: 156, SEQ ID NO: 168, SEQ ID NO: 180, SEQ ID NO: 192, SEQ ID NO: 204, SEQ ID NO: 216, SEQ ID NO: 228, SEQ ID NO: 240, SEQ ID NO: 252, SEQ ID NO: 264, SEQ ID NO: 276, SEQ ID NO: 288, and SEQ ID NO: 300.

8. The pharmaceutical composition of claim 1, wherein any one or more of the anti-CD300c antibody and an antigen-binding fragment thereof comprise:
(i) a heavy chain variable region, comprising:
CDR1 consisting of an amino acid sequence selected from the group consisting of SEQ ID NOS: 79, 115, and 221;
CDR2 consisting of an amino acid sequence selected from the group consisting of SEQ ID NOS: 80, 116, and 212; and
CDR3 consisting of an amino acid sequence selected from the group consisting of SEQ ID NOS: 81, 117, and 213; and
(ii) a light chain variable region, comprising:
CDR1 consisting of an amino acid sequence selected from the group consisting of SEQ ID NOS: 82, 118, and 214;
CDR2 consisting of an amino acid sequence selected from the group consisting of SEQ ID NOS: 83, 119, and 215; and
CDR3 consisting of an amino acid sequence selected from the group consisting of SEQ ID NOS: 84, 120, and 216.

9. The pharmaceutical composition of claim 1, wherein any one or more of the anti-CD300c antibody and an antigen-binding fragment thereof is selected from:
(i) a heavy chain variable region comprising CDR1 consisting of SEQ ID NO: 79, CDR2 consisting of SEQ ID NO: 80, and CDR3 consisting of SEQ ID NO: 81; and a light chain variable region comprising CDR1 consisting of SEQ ID NO: 82, CDR2 consisting of SEQ ID NO: 83, and CDR3 consisting of SEQ ID NO: 84;
(ii) a heavy chain variable region comprising CDR1 consisting of SEQ ID NO: 115, CDR2 consisting of SEQ ID NO: 116, and CDR3 consisting of SEQ ID NO: 117; and a light chain variable region comprising CDR1 consisting of SEQ ID NO: 118, CDR2 consisting of SEQ ID NO: 119, and CDR3 consisting of SEQ ID NO: 120; and
(iii) a heavy chain variable region comprising CDR1 consisting of SEQ ID NO: 211, CDR2 consisting of SEQ ID NO: 212, and CDR3 consisting of SEQ ID NO: 213; and a light chain variable region comprising CDR1 consisting of SEQ ID NO: 214, CDR2 consisting of SEQ ID NO: 215, and CDR3 consisting of SEQ ID NO: 216.

10. The pharmaceutical composition of claim 1, wherein any one or more of the anti-CD300c antibody or an antigen-binding fragment thereof comprise:
a heavy chain variable region comprising CDR1 consisting of an amino acid sequence represented by SEQ ID NO: 79, CDR2 consisting of an amino acid sequence represented by SEQ ID NO: 80, and CDR3 consisting of an amino acid sequence represented by SEQ ID NO: 81; and a light chain variable region comprising CDR1 consisting of an amino acid sequence represented by SEQ ID NO: 82, CDR2 consisting of an amino acid sequence represented by SEQ ID NO: 83, and CDR3 consisting of an amino acid sequence represented by SEQ ID NO: 84.

11. The pharmaceutical composition of claim 1, wherein any one or more of the anti-CD300c antibody and an antigen-binding fragment thereof comprise:
a heavy chain variable region comprising CDR1 to CDR3 consisting of amino acid sequences represented by the following formulas (1) to (3), respectively, and
a light chain variable region comprising CDR1 to CDR3 consisting of amino acid sequences represented by the following formulas (4) to (6), respectively:
(1) FTFX1X2X3X4MX5WVR
wherein,
X1 = G or S;
X2 = S, R, or D;
X3 = N or Y;
X4 = Y, A, G, or H; and
X5 = S or H,
(2) X1ISX2SGX3X4TYYAX5
wherein,
X1 = T or A;
X2 = G or S;
X3 = T or G;
X4 = S or Y; and
X5 = D or E,
(3) YCAX1X2X3X4X5X6X7X8X9W
wherein,
X1 = R or S;
X2 =G or S;
X3 = M, S, Y, or I;
X4 = W, Q, G, or R;
X5 = G or L;
X6 = M, I, or P;
X7 = D, F, or L;
X8 = V or D; and
X9 = I, Y, or absent,
(4) CX1X2X3X4X5X6X7X8X9X10X11VX12W
wherein,
X1 = T or S;
X2 = G or R;
X3 = K, N, or S;
X4 = H, N, or S;
X5 = R, I, or G;
X6 = H, G, or I;
X7 = T, I, or S;
X8 = R, A, K, or absent;
X9 = R, S, G, or absent;
X10 = N or absent;
X11 = Y or absent; and
X12 = N, H, or Q,
(5) X1X2X3X4RPSGVX5
wherein,
X1 = L, S, R, or E;
X2 = D, K, or N;
X3 = S or N;
X4 = E, N, Q, or K; and
X5 = P or R,
(6) YCX1X2X3X4X5X6X7X8X9X10VF
wherein,
X1 = Q, A, or S;
X2 = S or A;
X3 = Y or W;
X4 = D or A;
X5 = S, D, or G;
X6 = S, N, or T;
X7 = S, L, N, or K;
X8 = V, S, N, or G;
X9 = G, L, V, or absent; and
X10 = P or absent.

12. The pharmaceutical composition of claim 1, wherein any one or more of the anti-CD300c antibody and an antigen-binding fragment thereof recover or improve cognitive function in a subject having or at risk of a degenerative brain disease.

13. A method of preventing or treating a degenerative brain disease, comprising administering to a subject having or at risk of the degenerative brain disease any one or more of anti-CD300c antibody and an antigen-binding fragment thereof.

14. A food composition for preventing or treating a degenerative brain disease, comprising one or more of the anti-CD300c antibody and an antigen-binding fragment thereof as active ingredients.

15. A feed composition for preventing or treating a degenerative brain disease, comprising one or more of the anti-CD300c antibody and an antigen-binding fragment thereof as active ingredients.

16. Use of one or more of the anti-CD300c antibody or an antigen-binding fragment thereof for preventing or treating a degenerative brain disease.
